(19) Europäisches Patentamt
European Patent Office
Office européen des brevets

(11) Publication number: **0 273 774 A2**

# (12) EUROPEAN PATENT APPLICATION

(21) Application number: **87311532.3**

(22) Date of filing: **30.12.87**

(51) Int. Cl.$^4$: **C 12 N 15/00**
C 12 N 9/72, C 12 N 5/00,
C 12 P 21/02, A 61 K 37/54

(30) Priority: **30.12.86 US 947846 24.07.87 US 77586**

(43) Date of publication of application:
**06.07.88 Bulletin 88/27**

(84) Designated Contracting States:
**AT BE CH DE ES FR GB GR IT LI LU NL SE**

(71) Applicant: **CETUS CORPORATION**
**1400 Fifty-Third Street**
**Emeryville California 94608 (US)**

(72) Inventor: **Devlin, James Joseph**
**1146 Upper Happy Valley**
**Lafayette California 94549 (US)**

**Devlin, Patricia Egan**
**1146 Upper Happy Valley**
**Lafayette California 94549 (US)**

**Mark, David Fu-Chi**
**217 Stanbridge Court**
**Danville California 94526 (US)**

**Clark, Robin**
**3736 Woodruff Avenue**
**Oakland California 94602 (US)**

(74) Representative: **Bizley, Richard Edward et al**
**BOULT, WADE & TENNANT 27 Furnival Street**
**London EC4A 1PQ (GB)**

The applicant has filed a statement in accordance with Rule 28 (4) EPC (issue of a sample only to an expert). Accession number(s) of the deposit(s): ATCC 67280, 67279, 67431, 47432.

## (54) Plasminogen activators, DNA encoding the same and their preparation and use.

(57) Novel plasminogen activators are disclosed. Of the novel plasminogen activators, one is a modified urokinase that is altered to make it less susceptible to forming two chain-urokinase and to increase the fibrin specificity of urokinase. Also among the novel plasminogen activators are fibrinolytic proteins comprised of at least one domain capable of interacting with fibrin and the protease domain of urokinase. The domain capable of interacting with fibrin may have an amino acid sequences substantially the same as those of kringle-2 of t-PA, kringle-1 and/or -4 of plasminogen and the finger domain of t-PA. The protease domain may have an amino acid sequence substantially the same as that of 30 Kd urokinase. DNA sequences encoding the novel plasminogen activators, expression vectors and expression systems therefor are also disclosed and claimed.

EP 0 273 774 A2

## Description

# PLASMINOGEN ACTIVATORS, DNA ENCODING THE SAME AND THEIR PREPARATION AND USE

### Field of the Invention

The present invention relates to the field of molecular biology and proteins. In particular, it relates to novel proteins having the capacity to activate plasminogen, to methods for the production of such novel proteins, including the expression of such proteins using an insect cell/baculovirus expression system, and to various intermediates useful in the production of such novel proteins.

### Background of the Invention

The formation of blood clots in blood vessels of major organs in the body is one of the leading causes of human mortality in Western industrialized society. Myocardial infarction--heart attack--primarily caused by blood clot formation or thrombosis in the coronary artery, is the leading cause of death in the United States among adult males. Emboli, blood clots traveling in the circulatory system, which lodge in the blood vessels of the lung, brain, or heart are also significant causes of death in patients following surgery, dialysis and traumatic injury. Phlebitis, a condition in which thrombi, stationary blood clots, block circulation through the large blood vessels, particularly of the lower extremities, is also a serious life threatening disease.

The physiological mechanisms leading to blood clot formation are complex. Formation of the matrix of the blood clot involves the proteolytic activation of the circulating protein fibrinogen to form fibrin monomers. The fibrin monomers subsequently cross-link into a fibrin network. The events leading to the activation of fibrinogen to form fibrin are complex and are not completely understood; however, once fibrin monomers polymerize into threads, the fibrin threads form the reticulum of the growing blood clot. This fibrin thread reticulum enmeshes, and in some instances, binds platelets, blood cells and circulating plasma proteins to form the clot itself.

The mechanism of blood clot dissolution or fibrinolysis is also complex. At least three components are involved; plasminogen, plasminogen activators and plasmin inhibitors. Plasminogen is one of the circulating plasma proteins incorporated into a blood clot as it forms. Plasminogen is an inactive precursor or proenzymatic form of the protein plasmin, also sometimes referred to as fibrinolysin. Limited proteolysis of plasminogen yields plasmin, a proteolytic enzyme that digests fibrin threads, as well as other substances involved in the activation of blood clot formation such as fibrinogen, factor V, factor VIII, prothrombin, and factor XII. Plasminogen has a specific binding affinity for fibrin and thus a portion of the circulating plasminogen accumulates in the blood clot in association with the fibrin reticulum of the clot. After it is proteolytically activated, plasmin digests the fibrin reticulum of the clot and associated proteins as mentioned above. Plasminogen can be proteolytically activated to form plasmin by a number of enzymatically active proteins known as plasminogen activators.

Streptokinase, a protein released by streptococcal bacteria, is known to activate plasminogen to plasmin. It does not specifically bind to fibrin and as a result, it activates both circulating plasminogen and plasminogen in the blood clot. The plasmin so produced acts upon fibrin, as well as plasma proteins such as fibrinogen, factor V, and factor VIII, throughout the body. The generalized, rather than local manner in which streptokinase exerts its plasminogen activation, is a major drawback in its therapeutic use. Thus streptokinase, since it is enzymatically active against circulating plasminogen, can activate so much plasminogen that uncontrolled bleeding occurs in a patient.

The human plasminogen activators are classified in two groups: urokinase type plasminogen activators (urokinase or u-PA) and tissue type plasminogen activators (t-PA). These two classes of plasminogen activators can be distinguished immunologically, by tissue localization and stimulation of activity by fibrin.

Urokinase is initially produced as a single chain protein, pro-urokinase or as a single chain urokinase-type plasminogen activator (scu-PA) that can be proteolytically cleaved by plasmin to a 54000 dalton (54 Kd) molecular weight protein having two chains (tcu-UK). This two-chain 54 Kd protein can be further processed to form a 33 Kd low-molecular-weight form of tcu-PA. Kidney plasminogen activator has been suggested to be pro-u-PA. See Holmes et al., Cloning and Expression of the Gene for Pro-urokinase in Escherichia coli, Bio/Technology, 3:923-929 (1985) and Khono et al., Kidney plasminogen activator: a precursor form of human urokinase with high fibrin affinity see Bio/Technology, 2:628-634 (1984).

Several investigators have described the interaction between scu-PA and plasminogen. Lijnen et al., Activation of plasminogen by pro-urokinase I: Mechanism, J. Biol. Chem., 261:1253-1258 (1986). Collen D. et al., Activation of plasminogen by pro-urokinase II Kinetics, J. Biol. Chem., 261:1259-1266 (1986). The selectivity of 54 Kd scu-PA is greater for plasminogen associated with blood clots than the selectivity of two chain urokinase or two chain 33 Kd low-molecular-weight urokinase for plasminogen associated with blood clots. Gurewich et al., Effective and fibrin specific lysis by a zymogen precursor form or Urokinase (pro-urokinase) a study in vitro and in two animal species, J. Clin Invest., 73:1731-1739 (1979).

It has recently been shown that a 30 Kd form of scu-PA can be isolated from kidney cell cultures. Wijngarrds et al., Characterization and fibrin binding properties of different molecular forms of pro-urokinase from monkey kidney cell culture, Thromb. Res., Vol. 426, 749-761 (1986). The 30 Kd scu-PA lacks the kringle structure of 55 Kd scu-Pa, yet has specific fibrinolytic properties very similar to those of the higher molecular weight 55 Kd form of scu-PA. Rijken et al., Thrombosis Research, 47:761-768 (1986). Rijken et al. propose that 30 Kd scu-PA is a good candidate for recombinant expression, but no suggestion is made of workable cloning and expression means. Interestingly, although both 30 Kd and 55 Kd scu-PA molecule appear to specifically activate blood clot-associated plasminogen thereby exerting a specific fibrinolytic effect without consuming fibrinogen, they do not specifically bind to fibrin clots. Furthermore, scu-PA does not activate as much circulating plasminogen as is activated by sptreptokinase, apparently due to an inhibitor found in plasma. This inhibition however is reversed by fibrin, which forms the reticulum of the blood clot. Zamarron et al. Identification of a competitive inhibitor of pro-urokinase in human plasma, Thromb. Haemostasis, 52:27-30 (1985).

Two chain urokinase is similar to streptokinase in its pattern of activity. tcu-PA has a low binding affinity for fibrin but a high rate of proteolytic activation of both circulating and bound plasminogen. When scu-PA is cleaved after lysine 158 ($lys_{158}$) to form tcu-PA, a new amino terminal end is formed at $ile_{159}$.

Boning and Havsteen (J. Biol. Chem., 357(12): 6836-6843 (1982)) have suggested that an interaction between $asp_{194}$ and $his_{57}$ of chymotrypsinogen renders this protein inactive. They further suggest that after cleavage of this molecule in the region corresponding to the region containing $lys_{158}$ of scu-PA, the newly formed amino terminus forms an ion pair with $asp_{194}$, thereby reducing the interaction of $asp_{194}$ with the catalytic histidine residue and activating the protease. Although the crystal structure of scu-PA has not been elucidated, a similar process may occur during the conversion of scu-PA to tcu-PA. The cleavage after the $lys_{158}$ residue of scu-PA creates a molecule with less fibrin specificity than scu-PA. It would be desirable to make a scu-PA-like protease domain that is more fibrin specific than tcu-PA (because it is less susceptible to cleavage at the lysine residue corresponding to $lys_{158}$ of scu-PA) and is more active than some "noncleavable" forms of scu-PA (Nelles, et al. (J. Biol. Chem., 262(12):5682-5689 (1987)) have shown that a "noncleavable" form of scu-PA, made by converting $lys_{158}$ to $gly_{158}$, is less active than scu-PA) due to modifications that prevent the interaction between $asp_{355}$ (which corresponds to $asp_{194}$ of chymotrypsin) with the histidine at the active site.

Plasmin activated throughout the circulatory system of the patient by tcu-PA may lead to generalized and uncontrollable bleeding. tcu-PA is also inhibited by the same inhibitors as t-PA.

t-PA is produced by the cells lining the lumen of blood vessels or endothelial cells. t-PA is initially produced as a single chain protein. A two chain t-PA is formed by proleolytic cleavage of the single chain molecule into two polypeptides connected by a disulfide bond. Rijken, D. G., et al., J. Biol. Chem., 256:7035-7041 (1981). The heavy (H) chain of the two chain protein (molecular weight of approximately 30 Kd) is derived from the amino ($NH_2$)-terminal part of the single chain protein. The light (L) chain of the two chain protein (molecular weight approximately 28 Kd) is derived from the carboxy (COOH)-terminal region. Pennica et al., Nature 301:214-221 (1983).

The secondary structure of t-PA has also been described. In one current model of t-PA, the secondary structure or pattern of folding of the amino acid sequence making up the protein is composed of different structural domains associated with particular activities of the protein. Recombinantly produced L chain, for example, when separately expressed is capable of converting plasminogen into plasmin, but this activity is not accelerated by fibrin as is the case in the complete t-PA protein composed of H and L chain. Based on similarities in amino acid sequences, several distinct regions have been ascribed to the H chain: a finger domain which resembles a similar region of fibronectin that binds fibrin; a domain similar in amino acid sequence to human and mouse epidermal growth factor, and two kringle structures having amino acid sequences homologous to structural domains on plasminogen involved in the binding of fibrin to plasminogen. Kringle refers to a triple disulfide structure originally described in detail by Magnusson et al., Proc. Natl. Acad. Sci. USA, 72:2577-2581 (1975). The light chain has a serine protease region. t-PA specifically binds to fibrin and activates plasminogen entrained in the blood clot matrix to form plasmin. The enzymatic activity of t-PA increases measurably upon binding to fibrin. It is hypotheized that the binding of t-PA and plasminogen to fibrin, results in an advantageous alignment of the enzymatic portion of t-PA with its substrate plasminogen on the fibrin matrix which yields a localized production of plasmin, the primary clot-dissolving enzyme. The affinity of t-PA for fibrin furthermore is reported to be higher than that of u-PA or scu-PA. Thus, t-PA activates plasminogen in the blood clot, but has relatively low activity on circulating plasminogen. t-PA, however, has a short half-life in the patient, on the order of five minutes.

The structure of the portions of proteins having plasminogen activating activity has been the subject of a number of studies. Ichinose, et al. J. Cin. Invest., 78:163-169 (1986) carried out mild reduction and alkylation to separate the A (heavy) and B (light) chains of t-PA. The A chain was found to bind lysine suggesting that A chain also binds to fibrin and plasminogen. Further, fragmentation of the A chain with thermolysin yielded a fragment with the kringle region-1 of the A chain intact but kringle region-2 lysed. This fragment failed to bind lysine, whereas a somewhat larger fragment, in which both kringle-1 and -2 were apparently intact, bound lysine. The authors postulate that a region of kringle-2 is essential to t-PA binding of fibrin but observe that other segments of t-PA probably contribute to the maximum binding activity of t-PA.

European Patent Application 155,387 discloses hybrid fibrinolytically active proteins in which the A and B chains of the hybrid proteins are prepared by mixing one chain of one protease with a chain of another protease under oxidative conditions. The chains so mixed are disclosed as those of plasmin, t-PA and urokinase plasminogen activator. In the hybrid at least one of the chains is the fibrinolytically active chain of the above-mentioned fibrinolytic protease. The hybrid fibrinolytic protease is made by disulfide bond formation using the native sulfhydryl residues of the respective A and B chains used to make the hybrid protein. The A and B chains of the hybrid molecule however are not joined to one another as a continuous polypeptide chain. Although the disclosure mentioned that hybrid proteins may be prepared by taking the DNA sequence of each protein and constructing a new cDNA sequence coding for the hybrid protein, it fails to indicate any of the DNA sequences that can be used for this purpose, the nature of the protein so produced or expression system capable of expressing such hybrids or cells in which this task has been successfully accomplished.

Functional analysis of some of the structural domains of t-PA was reported by Van Zonneveld et al., Proc. Nat. Acad. Sci.,22(83):4670-4674 (1986). Investigation of the observed similarity of secondary structures lead to the postulation that the separate hypothetical domains harbor autonomous functions. Van Zonneveld et al. produced deletion mutants of the complete t-PA cDNA and expressed the mutants in cells. Depending upon the particular region deleted, functions of the remaining t-PA molecule were altered as determined by certain in vitro tests. In vitro, this assessment indicated that plasminogen activator activity is accelerated in the presence of fibrinogen in deletion mutants that contained the kringle-2 (K-2) region, and to a lesser extent those containing the finger (F) region and epidermal growth factor (E) region. Fibrin binding in in vitro assays also appeared to be associated with K-2-containing deletion mutants as measured by the ability of fibrin to compete with lysine analogues and to a lesser extent, mutants containing the E and F region. Furthermore, the authors established that an anti-human t-PA monoclonal antibody that partially inhibited fibrin binding to t-PA (isolated by McGregor et al., Thromb. Haemostatis, 53:45-50 (1985)), specifically bound to the t-PA deletion mutant containing only the L chain and K-2 region.

However, in constrast to results suggesting that fibrin binding resides chiefly in the K-2 region, a 96-base pair fragment that contained the E and F regions of t-PA competitively inhibited t-PA binding to fibrin. A. Klausner, Biotechnology, 4:709 (1986). Van Zonneveld et al., J. Biol. Chem., 261(30):14214-14218 (1986) separately showed that the finger region of t-PA also binds to fibrin but by a mechanism apparently different than that of the K-2 region. Furthermore, it is not clear that the activities of any of these regions is independent of the others. Alteration in one region may easily lead to unforeseen effects on other regions. Klausner, supra.

One of the drawbacks of t-PA is that although it has the ability to bind to fibrin in blood clots and to thus locally activate plasminogen in a blood clot, high therapeutic doses of t-PA can also cause enough systemic activation of plasminogen to cause the degradation of fibrinogen, the precursor to fibrin. Alteration of this fibrinogen degrading activity in t-PA would be desirable. Klausner, supra.

Another disadvantage of t-PA is that its ability to convert plasminogen to plasmin is strongly inhibited by a plasma-borne inhibitor. Several such inhibitors have been identified and isolated from extracts of human bood platelets and from a number of tumor cell lines, including the fibrosarcoma HT-1080. Andreasen et al., J. Biol. Chem., 261(17):7644-7651 (1986), and Klausner, supra. It has been reported that removal of the kringle regions of t-PA reduces such t-PA inhibitor effects in vitro, leading to speculation that t-PA could be made without an inhibitor binding site. However, authorities in this field have questioned whether such t-PA's without inhibitor binding sites would be biologically active. Klausner, supra.

tcu-PA also activates plasminogen to produce plasmin, but unlike t-PA, tcu-PA-mediated plasmin production is not localized to fibrin found in blood clots. tcu-PA's effect is systemic and generalized hemorrhagic events are a frequent side effect of its therapeutic use to break up local blood clots. tcu-PA is also inhibited by the same platelet- and HT-1080 produced-inhibitor as t-PA. By contrast, potential enzymatic activity of scu-PA is resistant to this inhibitor under conditions in which greater than 95% of the active u-PA forms a complex with the inhibitor. Andreasen et al., supra.

From the foregoing, it will be seen that each of the known plasminogen activators has drawbacks. Streptokinase and urokinase are both largely non-specific in their activation of both circulating and bound plasminogen. Tissue plasminogen activator has a higher specificity for fibrin-associated plasminogen but is not completely specific. Pro-urokinase appears to specifically activate blood-clot associated plasminogen thereby exerting a specific fibrinolytic effect without extensive consummation of circulating fibrinogen, however, like t-PA, at high therapeutic dosage some systemic activation of plasminogen occurs. Furthermore, both pro-urokinase and tissue plasminogen activators have numerous cysteine residues that must be paired to properly form disulfide bonds to assume the correct tertiary structure of the molecule when produced in a recombinant host. Tissue plasminogen activator has 35 cysteine residues and pro-urokinase is believed to have 24 cysteine residues.

It would be desirable to produce a protein that is specifically fibrinolytic. Preferably, such a protein would activate or consume circulating fibrinogen to a lesser extent than existing naturally occurring plasminogen activators. Optionally, the protein would be resistant to inhibition by platelet factors. Preferably, such a protein would be smaller than existing plasminogen activators such as t-PA and tcu-PA and scu-PA and would have fewer disulfide bonds. It would also be desirable for the new plasminogen activator to have a longer half-life than t-PA.

The challenges of expressing a modified scu-PA or tcu-PA protein by recombinant means and in particular, of producing a novel hybrid protein consisting of either a functional urokinase protease domain or a functional urokinase protease domain and at least one domain capable of interacting with fibrin, are numerous.

For example, limitations encountered with utilization of a number of host vector systems include the inability of prokaryotic host cells to perform post-translational modifications necessary for many eukaryotic proteins, the expense of mammalian cell culture media and difficulties with scale-up of mammalian host vector systems, and, with respect to yeast host systems, the instability of autonomously replicating yeast vectors and the differences in glycosylation patterns of the resulting protein. These difficulties are not insurmountable in all cases but merely indicate that some host-vector systems may be preferable for the expression of a particular gene construct.

The use of viruses in eukaryotic host-vector systems has been the subject of a considerable amount of recent investigation and speculation. However, some viral vector systems also suffer form significant disadvantages and limitations which diminish their utility. For example, a number of eukaryotic viral vectors are either tumorigenic or oncogenic in mammalian systems and create potentially health and safety problems associated with resultant gene products and accidental infection.

The baculovirus expression vector system involved in the instant invention overcomes many of the above-mentioned limitations. Baculoviruses are insect pathogenic viruses which, until recently, were studied mostly for their potential use as viral insecticides for control of agriculturally important insect pests.

Baculoviruses are unusual among animal viruses, not only in the protective function of the viral occlusion in the viral life cycle but also because the polyhedrin gene is the most highly expressed eukaryotic virus gene known. The polyhedrin protein can accumulate to greater than 1 mg/ml of infected cultured insect cells (70-75% of the total cellular protein) or can comprise up to 25% of the total protein of an infected insect. Although very highly expressed, neither the polyhedrin gene nor its protein is essential for viral infection or replication in cultured insect cells or insects, thus making the polyhedrin gene an ideal target for genetic manipulation.

The genetic engineering of the baculovirus polyhedrin gene for high level expression of a heterologous protein (recombinant human beta-interferon) was first reported by Smith, G.E., et al. (Mol. Cell. Biol.) 3(12):2156-2165 (1983). Since then, human interleukin 2 has been expressed in insect cells by a baculovirus expression vector as described by Smith et al. (Proc. Natl. Acad. Sci USA 82:8404-8408 (1985)). Recently, the synthesis of functional human T-cell leukemia virus Type I p40x protein using a baculovirus expression vector has been reported (Jeang, K.T., et al., J. Virol. 61:708-813 (1987)). Other heterologous proteins that have been expressed in this system are summarized in Summers et al. ("Genetic Engineering of the Genome of the *Autographa californica* Nuclear Polyhedrosis Virus" Banbury Report: Genetically Altered Viruses in the Environment 22:319-339, Cold Spring Harbor Laboratory (1985)).

The baculovirus expression system has several advantages for the expression of foreign genes in comparison to other prokaryotic, yeast or mammalian cell expression vector systems. First, high levels of expressed proteins are possible. Greater than 1.0 mg per ml of polyhedrin protein is normally produced in infected cells. Another non-essential occlusion-related viral protein named p10 is also abundantly produced and its promoter has reportedly been used to drive foreign gene expression (D.W. Miller et al. in Genetic Engineering Principles & Methods 8:277-298, Setlow and Hollaender, eds., New York: Plenum Press, 1986). Using the polyhedrin gene promoter, heterologous gene expression levels never reach polyhedrin levels but are usually in the range of tens to hundreds of micrograms per ml (Summers et al., (1985), p. 321 supra).

Secondly, in contrast to those produced in bacterial or yeast cells, recombinant proteins produced in insect cells may be co- and post-translationally processed in a manner similar to what occurs in mammalian cells. In at least one case, glycosylation of IFN-beta in infected insect cells has been reported (Smith et al., (1983) supra). Whereas about 40% of the natural IL-2 produced in human Jurkat cells is not glycosylated, there was no evidence of any glycosylation of the recombinant IL-2 produced in insect cells (Smith, et al., (1985) supra). In addition, correct cleavage of mammalian secretory signal peptides has been observed (Smith, et al., (1983) supra; Smith, et al., (1985) supra; Miller, et al., (1986) supra).

It would be desirable to produce the proteins described above that are specifically fibrinolytic by recombinant means using an insect cell/baculovirus expression system.

## Brief Description of the Invention

In one respect the present invention comprises a modified pro-urokinase that is incapable of forming two chain urokinase and whose activity is stimulated by fibrin to a greater degree than urokinase.

In this regard, the invention comprises a modified scu-PA wherein one modification includes deletion of lysine as position 158 ($lys_{158}$), the conversion of $lys_{158}$ into a neutral amino acid, or the insertion of at least one negatively charged amino acid within 5 amino acids of $lys_{158}$, of the conversion of at least 1 amino acid within 5 amino acids of $lys_{158}$ into a negatively charged amino acid.

A second modification prevents the association of the aspartic acid 355 with the histidine active site residue 204. The modification is obtained by placing a neutral or a positively charged amino acid residue within 5 amino acids of aspartic acid 355 or by substitution of amino acid residue 355.

One preferred embodiment of the invention provides a protein comprising a functional urokinase protease domain and at least one domain capable of interaction with fibrin.

Still another aspect of the invention concerns DNA sequences encoding the above-mentioned proteins.

A yet still another aspect of the invention concerns expression vectors which are capable of effecting the expression of the novel proteins, to the host cells transformed or infected with such vectors, and to the cultures thereof.

One preferred embodiment of the invention provides recombinant baculovirus transfer vectors in which the novel plasminogen activator proteins are encoded under the transcriptional control of a baculovirus polyhedrin gene promoter.

Still another aspect of the invention concerns a eukaryotic signal peptide sequence which is capable of effecting the secretion of the novel plasminogen activator proteins.

Also, aspects of the invention are methods for producing biologically active novel plasminogen activator proteins using susceptible host insect cells infected with recombinant baculovirus expression vectors.

In another aspect the invention includes the use of a protein of the invention in the manufacture of a medicament

These and other aspects of the invention will become more clear in conjunction with the following figures.

Brief Description of the Drawings

Figure 1 shows the deoxyribonucleotide sequences of oligodeoxyribonucleotides A-J used in construction of the DNA sequences according to the invention.

Figure 2 is a schematic representation of the formation of duplex DNAs from oligodeoxyribonucleotides A, B, C, and D and formation of plasmid pJD12.

Figure 3 is a schematic representation of the formation of duplex DNAs from oligodeoxyribonucleotides E, F, G, and H and the formation of plasmid pJD14.

Figure 4 is a schematic representation of the formation of duplex DNAs from oligodeoxyribonucleotides I and J and the formation of plasmid pJD13.

Figure 5 is a schematic illustration showing the manner in which plasmid pJD15 is formed.

Figure 6 is a schematic illustration of the steps required to modify restriction sites in the carboxyl terminal region of the coding sequence of the plasminogen activator and restriction sites 3′ thereto. Also shown are the steps taken to repair the DNA sequence encoding the novel plasminogen activator in a region 3′ to the NcoI site in plasmid pPD11 and the repaired plasmid pPD17 resulting therefrom. Plasmid pPD17 encodes a complete plasminogen activator according to the invention.

Figure 7 is a schematic representation of the steps required for formation of plasmid pPD7, pPD8 and pPD10, which are vectors for expressing the plasminogen activator according to the invention in E. coli, mammalian cells (COS), and insect cells (Spodoptera frugiperda), respectively.

Figure 8 is a schematic representation of the amino acid sequence of one embodiment of the novel plasminogen activator comprising the t-PA K-2 domain, scu-PA protease domain and a linker connecting the two domains. The numbering of the sequence does not correlate with either t-PA or scu-PA, thus to facilitate further discussions some general reference points are noted. Residues 1-83 comprise the K-2 domain of t-PA; residues 84-85 comprise a serine-threonine (ST) linker; and residues 86-349 comprise the scu-PA protease domain such that $lys_{158}$ of native scu-PA corresponds to the $gly_{96}$ of the depicted hybrid molecule.

Figure 9 is a Northern blot of polyA RNA of several cell lines using a urokinase-specific oligodeoxyribonucleotide probe. Significant pro-urokinase mRNA is detected in LD-1 and 5637 cells.

Figure 10 is a schematic representation of human urokinase. The arrows indicate cleavage sites. The arrow between $lys_{135}$ and $lys_{136}$ indicates the cleavage point that can yield a low molecular weight form of urokinase. The arrow between $lys_{158}$ and $ile_{159}$ indicates the cleavage point that yields tcu-PA.

Figure 11 is a schematic representation of human tissue plasminogen activator. The serine residue indicated by the broken arrow is the first residue of the mature protein according to Pennica et al., supra.

Figure 12 is a schematic representation of the protease domain of a form of the plasminogen activator according to the invention. The proteolytically labile lysine of the protease domain is indicated by a solid arrow. The aspartic acid residue, with which the $NH_2$ terminus formed by proteolysis at the labile lysine associates, is indicated by the broken arrow.

Figure 13 shows the amino acid and DNA sequence in a form of a plasminogen activator (PA-A) according to the invention. An ATG codon encoding an $NH_2$-terminal met residue may be included for expression in certain prokaryotic hosts.

Figure 14 shows the DNA sequence comparison between recombinant baculovirus transfer vectors pAcC1-C5. The carets indicate restriction endonuclease cleavage sites.

Detailed Description of the Invention

A. Cloning and Expression of the Novel Plasminogen Activator and Modifications of Pro-urokinase

The present invention is directed to novel proteins that have a number of the characteristics not found together in any plasminogen activating protein currently known. The proteins of the present invention may have the following characteristics separately or together.

In the following description and claims unless otherwise noted, the positions of amino acid residues in urokinase are according to the numbering of Holmes et al., Biotechnology 3:923-929 (1985) and the positions of amino acid residues in tissue plasminogen activator are according to the numbering of Pennica et al., Nature 301:214-221 (1983). Generally, the plasminogen activator hybrid molecules described herein are independently numbered. However, the correspondence among residues or nucleotides of the illustrated domains from t-PA, scu-PA or plasminogen and the present modified plasminogen activator molecules can be easily converted by those skilled in the art.

Upon proteolysis, scu-PA can form two chain urokinase in which the chains are joined by sulfhydryl linkage between cysteine residues. One chain has a molecular weight of ~20,000 and the other has a molecular weight of ~30,000. This 30 Kd chain of urokinase has the N terminal sequence

$NH_2$-ile ile gly gly glu phe thr . . . , resulting from cleavage between $lys_{158}$ and $ile_{159}$. See Kasai et al., Primary Structure of Single-Chain Pro-Urokinase, J. Biol. Chem. 260(22):12382-12389 (1985).

The enzymatic activity of scu-PA is known to be altered when it is processed at lysine residue 158 to form tcu-PA. In one respect, the present invention comprises a modified scu-PA wherein the modified protein is incapable of forming two chain urokinase. To make scu-PA that is incapable of forming tcu-PA, the amino acid sequence of scu-PA is altered in the region of $lys_{158}$. The alteration may take several forms. In one general approach according to the invention, $lys_{158}$ is converted to an amino acid that is resistant to proteolysis. In general, any amino acid that does not have a positively charged side group may be substituted for $lys_{158}$. Preferably, $lys_{158}$ is substituted by a neutral amino acid residue. By neutral amino acid is meant one that is non-charged at biocompatible pH (a range from about pH 6 to pH 8). Examples of such neutral amino acids included glycine, alanine and serine. In the present invention the change from $lys_{158}$ to $gly_{158}$ is demonstrated. Glycine, a non-charged amino acid, renders the site of cleavage of single chain scu-PA resistant to proteolytic attack.

An alternative approach to rendering this site resistant to proleolytic attack is the removal of $lys_{158}$ from the scu-PA molecule to make des-$lys_{158}$ urokinase. In a third approach to preventing the formation of tcu-PA, a negatively charged amino acid is placed within 5 amino acid residues in either the amino terminal direction or in the carboxyl terminal direction of the $lys_{158}$ residue. Such negatively charged amino acids are exemplified by aspartic acid and glutamic acid both of which are negatively charged at biocompatible pH (a range from about pH 6 to pH 8). The placement of the negatively charged amino acid residue may be accomplished by insertion between amino acids or substitution of an amino acid that occurs within 5 amino acids in the amino terminal direction or carboxyl terminal direction from $lys_{158}$. Without being bound by this theory, the inventors of the present invention believe that such negatively charged amino acids placed within the specified distance from $lys_{158}$ would render the $lys_{158}$ residue less prone to proleolytic attack due to interference with protease binding to the positively charged $lys_{158}$ residue.

It has been suggested, by analogy to the proteolytic cleavage of trypsinogen to trypsin, that after lysis of scu-PA to form tcu-PA, the positively charged new amino-terminus of the 30 Kd chain at $ile_{159}$ is associated with the negatively charged $asp_{355}$ residue of the two chain protein, by the formation of an internal ion pair between $ile_{159}$ and $asp_{355}$, leading to a conformation change. Kasani et al. supra.

Although the association of $ile_{159}$ with $asp_{355}$ and consequent change in conformation of the 30 Kd urokinase chain has only been suggested, the association may be disrupted either by inserting, between or substituting for, at least one of the amino acids comprising the $NH_2$ region of the 30 Kd chain, a positively or negatively charged amino acid. Such amino acids are exemplified by asp, glu, arg, and lys. Amino acids making up the N-terminal end of the 30 Kd chain witll generally be placed within 10 amino acid residue of the N-terminal $ile_{159}$, i.e., between and including $ile_{159}$ and $thr_{169}$. Preferably, they will be found between $ile_{159}$ and $phe_{164}$.

In another approach to increase the specificity of tcu-PA, according to the invention, $asp_{355}$ is deleted or changed to another amino acid that will disrupt the formation of an ion pair with the histidine active site residue at 204 of the protease domain.

The procedure for making scu-PA resistant to formation of the two chain species may be carried out by modifying a DNA sequence encoding scu-PA so that the codon encoding $lys_{158}$ of scu-PA is deleted or changed to one encoding a neutral amino acid sequence. Alternatively, the codons encoding amino acid residues within 5 amino acids of $lys_{158}$ may be modified to either substitute or insert a negatively charged amino acid.

Likewise, the modification to $asp_{355}$ of urokinase or amino acid residues within the region of this residue may be carried out by modifying a DNA sequence encoding scu-PA so that the codon encoding at least one of the specified amino acids is changed to a codon encoding a positively charged amino acid such as arg or lys, or encoding any of the neutral amino acids. Means for such insertions and substitutions of codons in DNA sequences are known and includes site specific mutagenesis using M13 vectors (see, e.g., Maniatis et al., infra).

DNA encoding scu-PA may be obtained by cloning from a number of sources. In the present invention as shown in detail hereinbelow, the cell line LD-1 was used. Total mRNA was obtained from cell line LD-1 and cDNA was created using AMV reverse transcriptase. The cDNA was cloned into a λgt10. The λgt10 library was screened using a probe designed to hybridize to a unique region of the DNA sequence encoding pro-urokinase. Lambda gt10 isolates that hybridized to the probe were expanded and the DNA enclodlng urokinase was isolated for further manupulation.

Although cell line LD-1 (Lilly and Rado, Blood 64:130a (1984)) was used herein, a number of other cell lines may also be used to obtain the DNA sequence encoding urokinase. The following cell lines yield DNA sequences encoding urokinase substantially identical to that obtained from LD-1: (1) Detroit 562 cells (ATCC No. CCL 138) see e.g. Jacobs et al., Molecular Cloning, Sequencing, and Expression in Escherichia coli of human prepro-urokinase cDNA, DNA:4(2):139-146 (1985) (amino acid differences occur at residue 366 glu to cys and 410 ala to val); Holmes et al., Cloning and Expression of the Gene for pro-urokinase in Escherichia coli, Biotechlogy3:923-929 (1985) Nagai et al., Molecular Cloning of cDNA coding for human prepro-urokinase, Gene, 36:185-188 (1985) (although some nucleotides differ, the amino acid sequence is the same); (2) cDNA library prepared from total RNA of human fibroblasts transformed by simian virus 40 (Okayama and Berg human cDNA library) see e.g. Verde et al., Identification and primary sequence of an unspliced human urokinase poly(A)+ RNA, Proc. Natl. Acad. Sci. USA, 80:4727-4731 (1984). (Again codons differ but only

amino acid residue $ile_{194}$ for $met_{194}$ differs.)

After the DNA sequence encoding pro-urokinase is obtained it may be modified at or within 5 amino residues of $lys_{158}$ and/or at $asp_{355}$ by several means including site-specific mutagenesis using an oligodeoxyribonucleotide encoding the desired sequence which is used to modify the urokinase DNA sequence in the region encoding the $lys_{158}$ residue and/or $asp_{355}$ residue.

Alternatively, the desired sequence may be produced as an oligodeoxyribonucleotide, amplified by cloning into a cloning vector, removed from the cloning vector and cloned into the DNA sequence encoding urokinase using available restriction sites in the urokinase DNA sequence that border the $lys_{158}$ and/or $asp_{355}$ residue. If desired, such oligodeoxyribonucleotides may be amplified entirely in vitro using the polymerase chain reaction method disclosed in Saiki et al., Science, 234:1530-1534 (1985).

As mentioned above, the complete amino acid sequence of pro-urokinase has been determined. See, e.g., Holmes et al., supra or Jacobs et al., supra. A DNA sequence encoding urokinase, with the alterations to or in the region within 5 amin acid residues of $lys_{158}$, and/or $asp_{355}$ can be constructed by the synthesis of overlapping oligodeoxyribonucleotides of various lengths until the complete coding sequence of the modified urokinase is formed. Chemical means for such oligodexoyribonucleotide DNA synthesis are readily available in automated processes sold by a number of commercial sources.

Urokinase as used herein is a general term that includes both scu-PA and tcu-PA-like plasminogen activators.

scu-PA as used herein means a protein having a single amino acid chain and the characteristics of activating blood clot-associated plasminogen thereby exerting a more specific fibrinolytic effect than tcu-PA. scu-PA is also known as pro-urokinase. Two forms of scu-PA having these activities are known, one having a molecular weight of ~55,000 (55 Kd) and the other a molecular weight of ~30,000 (30 Kd). See Rijken et al., Thrombosis Research, 47:761:763 (1986). Proteins having these activities have amino acid sequences as disclosed by Holmes et al., Jacobs et al., Verde et al., and Nagai et al., supra. As is clear from these sources, scu-PA may vary in primary amino acid sequence without loss of the fibrin-selective characteristics of the protein. The improved scu-PA according to the ivention has the characteristic of having a stable single chain form. This stability is manifested in a resistance to lysis of scu-PA at lysyl residue 158. In addition, the improved scu-PA has increased specificity for fibrin. This characteristic is accomplished through the disruption of ion bond formation between the aspartic acid residue 355 and the histidine active site residue 204. Thus, scu-PA within the scope of the invention may be considered to be any scu-PA or derivative thereof that has been rendered resistant to cleavage at residue 158 and will not form an ion bond with the active site histidine, and has the above-mentioned activities. In the 55 Kd form of scu-PA, the protein will be resistant to formation of tcu-PA.

As is the case for all proteins, the precise chemical structure of scu-PA, tcu-PA or the modified plasminogen activators of the invention depends on a number of factors. As ionizable amino and carboxyl groups are present in the molecule, the protein may be obtained as an acidic or basic salt, or in neutral form. All such preparations which retain their activity when placed in suitable environmental conditions are included in the definition of urokinase. Further, the primary amino acid sequence may be augmented by derivatization using sugar moieties (glycosylation) or by other supplementary molecules such as lipids, phosphate, acetyl groups and the like, more commonly by conjugation with saccharides. The primary amino acid structure may also aggregate to form complexes. Certain aspects of such augmentation are accomplished through post-translational processing systems of the producing host; other such modification may be introduced in vitro. Further, individual amino acid residues in the chain may be modified by oxidation, reduction, or other derivatization. Such alterations which do not destroy activity do not remove the protein sequence from the definition.

Modifications to the primary structure itself by deletion, addition, or alteration of the amino acids incorporated into the sequence during translation can also be made without destroying activity of the protein. Such substitutions or other alterations result in proteins having an amino acid sequence which falls within the definition of proteins "having an amino acid sequence substantially equivalent to that of urokinase".

Another primary aspect of the invention relates to novel plasminogen activators which are hybrids between a urokinase protease domain and at least one domain capable of interacting with fibrin. These modified plasminogen activators are characterized by having more specific fibrinolytic activity compared to scu-PA through the addition of a domain capable of interacting with fibrin. In an alternate embodiment the protein also is resistant to inhibition by platelet factors. In a preferred embodiment the protein activates less unbound plasminogen than scu-PA and as a result does not cause as much consumption of fibrinogen by systemically activated plasmin.

By "plasminogen activator" is meant a protein that converts the protein plasminogen to the active form plasmin. Such proteins may take a variety of hybrid forms including those created by 1) combining regions of more than one plasminogen activator molecule such as scu-PA, tcu-PA, t-PA or streptokinase to result in a protein having plasminogen activator activity; 2) combining regions of a plasminogen activator molecule such as those mentioned above with at least one protein domain capable of interacting with fibrin such as the kringle-2 of t-PA, the kringle-1 of plasminogen or the finger region of t-PA to result in a protein having plasminogen activator activity; 3) including in the combinations of 1) or 2) above at least one synthetic linker peptide region connecting protein domains to result in a protein having plasminogen activator activity; and 4) truncation, deletion or modification of the primary sequence of any of the molecules described in 1), 2) or 3) above to result in a protein having plasminogen activator activity. Modifications to the primary structure itself by deletion, addition, or alteration of the amino acids incorporated into the sequence during translation can be made without destroying the activity of the protein.

Plasminogen activation is shown by a number of assays. The fibrin plate assay is one such assay. See J. Ploug et al., Urokinase: an activator of plasminogen from human urine isolation and properties, Biochim. Biophys. Acta, 24:278-282 (1952). The plasma clot lysis assay described in Zamarron et al., Throm Haemostatis, 52:19-23 (1984), may also be used. In this assay, plasma clots are prepared by mixing 1 ml pooled normal plasma, 25,000 cpm $^{125}$I-labeled human fibrinogen, 50 µl 0.5 M CaCl and 100 µl of 80 NIH U/ml thrombin. The mixture is immediately drawn into a 4 mm internal diameter plastic tube and incubated at 37° C for one hour to allow clotting and cross linking. The clot is removed from the tube, washed in 0.15 M NaCl, pH 7.4, and cut into ~1.0 mm pieces. Each piece is counted in a gamma counter, and incubated in a 5 ml tube containing 2.45 ml pooled citrated normal plasma. Varying amounts (50-200 U/ml) of the plasminogen activator to be tested are suspended in 0.15 M NaCl, pH 7.4. Plasma samples are taken at intervals and released $^{125}$I fibrin degradation products are counted.

Extracts from recombinant cells may be assayed for plasminogen activation according to the method described in Pennica et al., supra. Briefly, an aliquot of cell extract containing plasminogen activator is incubated at 37° C for 10 minutes in 0.15 ml of 50 mM Tris-HCl pH 7.4, 12 mM NaCl containing 0.37 mg/ml plasminogen and 2.6 mg/ml fibrinogen. 0.35 ml of a 1 mM solution of the chromogenic substrate S-2251 (Kabivitrum Molndal, Sweden) in the above buffer is added and the reaction is continued for 5-30 minutes at 37° C. Acetic acid is added to a final concentration of 0.18 M to stop the reaction. Samples are centrifuged and absorbance at 405 nM is measured. Units are determined by comparison to a urokinase standard.

Plasminogen activation activity may also be determined on Casein/agarose plates containing plasminogen, as described in Saksela, O., Analytical Biochem, 111:276-282 (1981). Briefly, petri dishes are prepared with an agarose medium containing 100 ml of medium, which contains 37.5 ml $dH_2O$, 10 ml 10x phosphate buffered saline, 40 ml 2.5% agarose, 12.5 ml of 8% boiled powdered milk and 100 µg of plasminogen (Calbiochem #528175) in 20 mM Tris-HCl, pH8. The boiled powdered milk is prepared by boiling an 8 g/100 ml $dH_2O$ solution of milk for 20 minutes; centrifuging 20 min at 10,000xg and retaining the supernatant. The liquid agarose solution is added at 65° C; plasminogen is added when the mixture has cooled, but not hardened. 20 ml is added to a 100 mm Petri dish. To assay plasminogen activation, 20 µl of solution containing a plasminogen activator is placed in 4 mm holes punched in the casien agarose, and incubated at 37° C for 2-24 hours. The radius of the lysis halo that forms as the casien is degraded by plasmin is in proportion to the log of the amount of plasminogen activator being that was placed in the well. A standard plasminogen activator, such as urokinase, is used to calibrate the assay.

By "fibrinolytic activity" it is meant that the protein is able to the activate plasminogen to cause lysis of fibrin in one of the above-indicated assays. The specificity of the fibrinolytic activity possessed by the plasminogen activator according to the invention is defined by a lower level of proteolysis (compared to urokinase) of other proteins involved in clot lysis or in clot formation, particularly the activation of plasminogen and fibrinogen that are not bound in blood clots. Fibrinogen degradation may be determined by the method of Clauss et al., Acta Biochem. Biophys., 17:237-246 (1957).

The hybrid plasminogen activator molecules of the present invention were constructed to augment the specificity of the fibrinolytic activity of known plasminogen activators such as scu-PA. As stated previously, one of the disadvantages associated with tcu-PA concerns its non-specific activation of both circulating and bound plasminogen. While scu-PA appears to specifically activate blood-clot associated plasminogen, it has little affinity for fibrin. In contrast, t-PA has the ability to bind fibrin in blood clots and to thus locally activate plasminogen in a blood clot. Several domains of t-PA and plasminogen have been demonstrated to be associated with fibrin binding. Combinations of these domains, which are capable of interacting with fibrin, with the urokinase protease domain will augment the specificity of fibrinolytic activity of these modified plasminogen activators by possessing the property of locally activating plasminogen at the clot sites. This latter property is what is meant by the phrase "capable of interacting with fibrin," with reference to the domains of t-PA and plasminogen used to construct the hybrid plasminogen activators of the present invention.

Thus, given the presence of the fibrin binding domains that are capable of interacting with fibrin in these modified plasminogen activators, the hybrid molecules will exhibit a higher degree of interaction with fibrin than the native urokinase-type molecules. This interaction can be demonstrated with fibrin binding or fibrin stimulation assays.

One means for direct assessment of fibrin activity can be made using a spectrophotometric solid phase fibrin tissue-plasminogen activator activity assay essentially as described in Angles-Cano, E., Analytical Biochemistry, 153:201-210 (1986). This assay described extensively in international patent application publication WO 85/04425, published October 10, 1985, allows the specific and exclusive assessment of plasminogen activators with high affinity for fibrin and is based on the affinity separation of high-fibrin affinity plasminogen activators by a solid phase fibrin network. Briefly, fibrinogen is bound to wells of a polyvinyl chloride substrate plate with glutaraldehyde followed by the addition of a human thrombin and washing the plates. A bound fibrin network results. Fibrin specific plasminogen activator is placed in binding buffer and added to the plate. After washing unbound protein, fibrin-specific plasminogen activator protein is determined by measuring the generation of plasmin as follows. Plasminogen in a solution containing the chromogen S2251 is added and incubated, and absorbance at 405 nm is determined. Binding affinity is determined by adding various concentrations of plasminogen activator to plates in the presence of substrate solution and determining absorbance over a time course. Total fibrin-specific plasminogen activator activity is measured using a liquid phase system as follows. An aliquot of plasminogen activator is added to 50 μl per well of substrate solution containing an aliquot of purified fibrin in a fibrin bound plate. The change in absorbance at 405 nM per minute of fibrin bound plasminogen activator is used to calculate the fractional enzyme binding according to the equation

$$Fn/CM^2 = \frac{q}{S} = \frac{FnVa}{S}$$

where q = Ptα is the concentration of PVC-bound fibrin (mol), α is the partition coefficient of the fibrinogen adsorption procedure, S is the well surface area, and pt is the total fibrinogen concentration per volume. The dissociation constant Kp of the plasminogen activator, and hence the effectiveness of binding of the plasminogen activator may be calculated by combining the mass balance equation for the fibrin plasminogen binding equilibrium reaction:

$$\frac{[Fn] \quad [PA]}{[Fn.PA]} = Kp = \frac{MP}{9}$$

with the above equation and with the equation relating to mass conservation of plasminogen activator and fibrin molecules: mt = m + q and pt = p + q where mt is the total concentration of fibrin and pt is the total concentration of plasminogen activator; i.e.,

$$Kp = \frac{(mt-pt\alpha)\ (pt-pt\alpha)}{pt\alpha}$$

The fibrin binding properties of plasminogen activators may also be determined according to the methods described in Lostkutoff et al., Blood, 62:62-68 (1983).

Plasminogen activators having high affinity for fibrin have affinities expressed as Kp in a range approximately that of tissue plasminogen activator which has a Kp of 1.4 ±2 nM as determined in the above-identified assay. See Angles-Cano, supra. High affinity for fibrin in this assay is in a range of 1-2 nM.

Anther method used to indirectly assess the interaction of the hybrid plasminogen activator with fibrin is through the use of a fibrin stimulation assay. Typically, fibrin stimulation is determined by incubating the modified plasminogen activator with human plasminogen and a plasmin chromogenic peptide, in the presence or absence of fibrin. If the addition of fibrin causes more than a twofold increase in the amount of plasmin produced (fibrin generally causes less than a twofold increase in plasmin produced by native urokinase), then the modified plasminogen activator is said to exhibit increased interaction with fibrin. Details of this assay are provided in the accompanying examples.

By "resistance to inhibition by platelet factors" is meant that activity of the plasminogen activator according to the invention is not inhibited by the inhibitor identified in human blood platelet extracts and shown to be identical to the inhibitor produced by fibrosarcoma HT1080 in the above-indicated assays. See Anderson et al., J. Biol. Chem., supra. The resistance to inhibition by platelet factors is distinct from the reversible inhibition of scu-PA caused by inhibitors found in plasma; this reversible inhibition can be removed by cyanogen bromide-digested fibrinogen or fibrin. See Lijnen, et al., J. Biol Chemistry, supra.

The amino terminal end of the novel protein according to the invention comprises at least one domain that is capable of interacting with fibrin. In all of the embodiments of the present invention, the amino terminal end of the protein is made by creating a DNA sequence encoding the amino terminal end of the protein, ligating the DNA sequence into an appropriate expression vector and transforming a host to express the protein encoded by the DNA sequence. In one embodiment of the invention, a DNA sequence encodes a protein having at least the following amino acid sequence:

$NH_2$ G C Y F G N G S A Y R G T H S L T E S
G A S C L P W N S M I L I G K V Y T A Q N
P S A Q A L G L G K H N Y C R N P D G D A
K P W C H V L K N R R L T W E Y C D V P S
C S T C G Q K T L R P R F G I I G

The letter abbreviations for amino acids are those recommended by the IUPAC-IUB Commission on Biochemical Nomenclature, Dayhoff, M.V., Atlas of Protein Sequence and Structures, Vols. 5..Suppl. 3, Nat. Biomedical Res. Foundation, Washington, D.C. 1978.

The DNA sequence encoding the carboxyl end of the above amino acid sequence also encodes a linker sequence which ends in an EcoRI site that encodes the amino acids glutamic acid and phenylalanine when the nucleotide sequences are ligated to an EcoRI restricton site spanning amino acids 163 and 164 of pro-urokinase.

Also within the scope of the invention are plasminogen activators in which the above-indicated 99 amino acid sequence has been altered, by substitution deletion or insertion of amino acids but retains the ability to interact with fibrin to a greater extent than urokinase. Preferably, the location of the cysteine residues will be undisturbed so that the intra-chain disulfide bonds between cysteine residues are not disturbed. Furthermore, the residues $asp_{57}$, $arg_{70}$ and $trp_{74}$ as numbered will in Figure 8 may be conserved in such variant molecules as they appear to be required for the binding of the plasminogen activator.

Other amino acid sequences that interact with fibrin may be substituted for or joined to the portion of the above-indicated plasminogen activator that form the aforementioned kringle structure, i.e., the 80 amino acids beginning with the first cysteine residue and ending with the sixth cysteine residue in the sequence of Figure 8. Kringles 1 and 4 of plasminogen have been shown to bind lysine and to be displaced from binding to fibrin by the lysine analog epsilon-amino caproic acid (EACA). Kringle region 1 or 4 of plasminogen furthermore have similarly positioned amino acids involved in the binding of lysine; in kringle-4 of plasminogen $asp_{57}$, $arg_{71}$ and $trp_{72}$ appear to be involved in lysine binding. A similar arrangement of amino acids is present in kringle-1 of plasminogen. See generally Van Zonneveld et al. on the interaction of the finger and the kringle-2 domain of tissue-type plasminogen activator with fibrin, J. Biol. Chem., 261(80):14214-14218 (1986). Thus, within the scope of the invention are plasminogen activators wherein the amino acid sequence of kringle regions 1 or 4 of plasminogen, or both, are either joined to the kringle region of the above-indicated plasminogen activator, or are substituted therefor.

Also within the scpe of the invention are plasminogen activators in which the amino acid sequences of K1 and K4 of plasminogen may be altered by substitution, deletion or insertion of amino acids but retains the ability to interact with fibrin. Preferably cysteine residues are substantially conserved and the positions of $asp_{57}$, $arg_{71}$ and the aromatic amino acid at position 72 of the plasminogen activator kringles may also be conserved. The amino acid sequence for plasminogen is known (Sottrup-Jensen, L., et al., Prog. Chem. Fibrinolysis Thrombolysis, 3:191 (1978)). DNA sequences encoding the appropriate kringle regions of plasminogen may be constructed by known means either by oligonucleotide synthesis or by cloning of a DNA sequence encoding plasminogen from cell lines known to produce this protein.

Also among the other amino acid sequences that interact with fibrin that may be substituted for or joined to the above-indicated sequences which form the various kringle regions of the plasminogen activator, is an amino acid sequence comprising the so-called finger region of t-PA. The finger region of t-PA is within amino acids 1-50 of t-PA as numbered by Pennica et al., supra. DNA encoding this amino acid sequence may be synthesized as oligodeoxyribonucleotides by conventional synthesis means or may be cloned as a fragment of the cDNA constructed from any of the available t-PA producing cell lines or known recombinant plasmids carrying the t-PA DNA sequence.

The DNA encoding the domains capable of interacting with fibrin may be joined, to one another, if desired, by the addition of commercially available linkers to the ends of the coding sequences. The DNA sequences may also be joined by blunt-end or sticky end ligation after preparation of appropriate sticky ends, as is known in the art.

The choice of linkers may depend upon restriction endonuclease requirements of spatial considerations specific to each hybrid construction. Such linkers may be based on the DNA encoding the natural amino acids present immediately upstream of the protease domain of urokinase, immediately downstream or upstream of the respective kringle domains of plasminogen or t-PA, immediately downstream of the finger domain in t-PA, or a "hybrid" sequence among any of the aforementioned sequences, or the linker may include a synthetic sequence. Illustrative linker sequences are provided below:

$NH_2$-ST
$NH_2$-SGSGPPPPPPSGSGPPPPPPSGSG
$NH_2$-ADGGGPSSPPEELKFQ
$NH_2$-ADGKKPSSPPEELKFQ
$NH_2$-EEEPSSPPEELKFQ

The DNA sequence encoding the amino terminal end of the plasminogen activator according to the invention may be made in a number of ways. The DNA sequence encoding a number of the domains capable of interacting with fibrin of the present invention may be made as a single oligodeoxyribonucleotide sequence or as a series of overlapping oligodeoxyribonucleotide fragments that are ligated together by known means to yield the desired fragment. As exemplified in one embodiment of the invention, as an alternative method, part of the DNA sequence can be obtained by selective restriction endonuclease digestion of a DNA sequence including the region encoding the K-2 domain of t-PA The K-2 region corresponds to amino acids 180-263 and nucleotides 727-928 of the t-PA sequence reported by Pennica, et al. DNA encoding t-PA may be isolated according to the methods disclosed in Pennica et al., or from, for example, E. coli K12 strain 294 (ATCC No. 31446), an E. coli transformant having a recombinant DNA sequence encoding t-PA.

Furthermore, DNA encoding t-PA is obtainable from a variety of cell lines that are publicly available, including, for example, the Bowes Melanoma cell line, Wilson et al., Cancer Research, 40:933-938 (1980). The DNA sequence so obtained may be modified to remove extra or unwanted deoxyribonucleotides by site specific mutagenesis, endonuclease digestion followed by gel electrophoresis, S1 nuclease digestion or by Bal1 exonucleolytic digestion. (See generally Maniatis et al., infra).

Site specific mutagenesis may be used to insert a glycine codon immediately 5' of the codon encoding the cysteine residue closest to the $NH_2$-terminus of the protein according to the invention shown in Figure 8. Synthetic oligodeoxyribonucleotides may be constructed to furnish any part of the sequence as desired. A combination of isolation of the DNA encoding t-PA from any of the above-mentioned sources, together with site specific mutagenesis and ligation to oligodeoxyribonucleotides to encode the desired sequence may be used.

Other definitions used for the expression of the novel plasminogen activators according to the present invention include the following.

"Operably linked" refers to juxtaposition such that the normal function of the components can be performed. Thus, a coding sequence "operably linked" to control sequences refers to a configuration wherein the coding sequence can be expressed under the control of these sequences.

"Control sequences" refers to DNA sequences necessary for the expression of an operably linked coding sequence in a particular host organism. Eukaryotic cells including the insect cells of the instant invention appear to utilize promoters and polyadenylation signals.

"Expression system" refers to DNA sequences containing a desired coding sequence and control sequences in operable linkage, so that hosts transformed with these sequences are capable of producing the encoded proteins. These DNA sequences may also direct the synthesis of the encoded proteins in an in vitro cellular environment. In order to effect transformation, the expression system may be included on a transfer vector; however, the relevant DNA may then also be integrated into the viral chromosome to result in a recombinant viral genome.

As used herein, "cell", "cell line", and "cell culture" are used interchangeably and all such designations include progeny. Thus "transformants" or "transformed cells" includes the primary subject cell and cultures derived thereform without regard for the number of transfers. It is also understood that all progeny may not be precisely identical in DNA content, due to deliberate or inadvertent mutations. Mutant progeny which have the same functionality as screened for in the originally transformed cell are included. Where distinct designations are intended, it will be clear from the context.

"Infection" as used herein refers to the invasion of cells by pathogenic viral agents where conditions are favorable for their replication and growth. "Transfection" refers to a technique for infecting cells with purified nucleic acids by adding calcium chloride to solutions of DNA containing phosphate or other appropriate agents such as dextran sulfate thereby causing the DNA to precipitate and be taken up into the cells.

"Recombinant transfer vector" refers to a plasmid containing a "heterologous" gene under the control of a functional promoter (e.g., polyhedrin p10, ot trp promoters) and flanked by viral or bacterial sequences according to the respective host to be used. The "recombinant expression vector" or "recombinant virus" is formed after transformation of bacterial or mammalian host cells with the recombinant transfer vector or, in the embodiment using viral vectors, after cotransfection of wild-type baculovirus DNA into host insect cells whereupon homologous recombination occurs between the viral sequences flanking the heterologous gene and the homologous sequences in the wild-type viral DNA. Recombination results in the incorporation of the heterologous gene and flanking control sequences into the virus. The recombinant expression vector is the recombinant viral DNA containing the desired heterologous gene.

"Biologically active" refers to retaining the enzymatic function of the protein. The biological activity of plasminogen activator is the conversion of plasminogen to the active form plasmin.

"Secretory signal peptide" refers to a sequence of amino acids that causes a protein expressed in insect cells, such as plasminogen activator or modified plasminogen activators, to be secreted outside the cell. A "heterologous secretory signal peptide" is an amino acid sequence not naturally found in association with the protein to be secreted. Signal peptide as used herein refers to not just the amino terminal peptide that is cleaved to generate the mature protein but also to residues +1 up to +5 that may have been included for either convenience of construction or to help direct the site of cleavage.

B. General Methods for Carrying out the Invention

Transformations

Depending on the host cell used, transformation is done using standard techniques appropriate to such cells. The calcium treatment employing calcium chloride, as described by Cohen, S. N., Proc. Natl. Acad. Sci. (USA), 69:2110 (1972), or the RbCl method described in Maniatis, et al., Molecular Cloning: A Laboratory Manual (1982) Cold Spring Harbor Press, p. 254, was used for prokaryotes or other cells which contain substantial cell wall barriers. For mammalian cells without such cell walls, the calcium phosphate precipitation methods of Graham and van der Eb, Virology, 52:546 (1978), or Wang et al., Science, 228:149 (1985), may be used. The DEAE dextran method of Manos and Gluzman, Mol Cell Bio, 4:1125-1133 (1984), is also useful for mammalian cell transformations. Transformations into yeast are carried out according to the method of Van Solingen, P., et al., J. Bact., 130:946 (1977) and Hsiao, C. L., et al., Proc. Natl. Acad. Sci. (USA), 76:3829 (1979).

## Screening cDNA Libraries

cDNA libraries are screened using the colony hybridization procedure. Lifts of colonies are made onto nitrocellulose filter papers (S & S type BA-85). The colonies are lysed and DNA fixed to the fiter by treatment for five minutes with 0.5 M NaOH, 1.5 M NaCl, and are then washed twice for five minutes each time with 1.0 M Tris pH 8, 3 M NaCl. Filters are air dried and baked at 80°C for two hours. The duplicate filters are prehybridized at 45-50°C for one hour in 5 x SSC, 10 x Denhardt's solution (0.2% polyvinylpyrrolidone, 0.2% Ficoll, 0.2% BSA), 0.1% SDS, 50 mM sodium phosphate pH 7.0, and 100 µg/ml tRNA.

Hybridization of the filters is done in a solution similar to that described above for prehybridization, but also contains 10% dextran sulfate with kinased probe at 1 x $10^6$ CPM/ml under conditions which depend on the stringency desired. Typical moderately stringent conditions employ temperatures of 45-50°C for 16-20 hours with 1-5 ml/filter of DNA hybridization buffer containing probe. For higher stringencies, higher temperatures are employed. The filters are washed three times for 15 minutes each time at appropriate temperatures using 3 x SSC, 0.1% SDS, air dried, and are autoradiographed at -70°C for two to three days.

## Design of a Eukaryotic Consensus Secretory Signal Peptide Sequence

One approach to obtaining modified plasminogen activator secreted from, in this case, Sf-9 insect cells and CosA2 cells, a mammalian cell line, was to design a eukaryotic secretory signal sequence based on consensus usage of amino acids at particular positions included in either the 161 eukaryotic or the 36 prokaryotic non-homologous (depending on the intended host cell) signal sequences comprising the database used by von Heijne (Nucl. Acids Res., 14:4683 (1986) ; von Heijne, G., J. Mol. Biol., 184:99 (1985)). Such a consensus secretory signal peptide is expected to be capable of directing the secretion of heterologous proteins other than modified plasminogen activators such as lymphokines, including colony stimulating factors, interleukin-2 or interferons, other hematopoetic factors, hormones, antigens or growth factors. In addition such a consensus secretory signal peptide is expected to function in eukaryotic cell lines other than Sf-9 insect cells such as monkey COS-M6 cells, Chinese hamster ovary cells, CV-1 cells, L cells and myelomas.

As disclosed herein, secretory signal peptide sequences may be designed using the following reasoning. G. von Heijne (1985 and 1986, supra) has suggested that a comparison of the 13 amino acid residues preceding (-13 to -1 positions) and the 2 amino acid residues (+1 and +2 positions) following the cleavage site in known signal peptides can be used to predict the cleavage site for other secreted proteins. The amino acid residues most frequently found in these 15 positions in the database have been summarized (von Heijne, supra). Using these tables showing the most frequently used amino acid residues in signals peptides, it is possible to design a signal peptide by choosing amino acid residues that are among a group of residues must frequently found at each position in natural signal sequences. The most frequently used residues can be chosen either by correcting for the amino acid composition of the database (which includes mature as well as secretory peptide sequences) or by selecting the most frequently used residues without correcting for the prevalence of a given amino acid in the database. Since there is obviously some variation in nature among secretory peptide sequences, it is not necessary to pick the most frequently used residue for each position. In other words, residues that are not the most frequently used can be selected for some of the positions.

The amino terminal residues in the signal peptide were not found to be predictive for the cleavage site (von Heijne, G., supra). Therefore, the amino terminal residues of the synthetic signal peptide can be chosen either based on the consensus method described above or by using the appropriate portion of a naturally occurring signal peptide.

It is also possible that greater than or fewer than the suggested 15 residues may be chosen by the consensus method and still produce a synthetic signal sequence that is cleaved in the predicted position. This method may be used for both eukaryotic or prokaryotic expression systems using in each case the appropriate database for predicting the consensus residues.

## Vector Construction

Construction of suitable vectors contianing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solutions. In the examples herein, typically, a 3-10 fold excess of restriction enzyme is used to insure complete digestion of the DNA substrate. Incubation times of about one hour to two hours at 37°C or other appropriate temperatures are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separations is found in Methods in Enzymology, 65:499-560 (1980) or Maniatis, Molecular Cloning: A Laboratory Manual, Cold Spring Harbor Laboratory, Cold Spring Harbor, NY (1982).

Restriction cleaved fragments may be blunt-ended by treating with the large fragment of E. coli DNA polymerase I (Klenow) in the presence of the four deoxynucleotide triphosphates (dXTPs) using incubation times of about 15 to 25 minutes at 20 to 25° C in 50 mM Tris pH 7.6, 50 mM NaCl, 6 mM $MgCl_2$, 6 mM DTT and 5-10 µM dXTPs. The Klenow fragment fills in at 5′ overhangs but chews back protruding 3′ single strands, even though the four dXTPs are present. If desired, selective repair can be performed by supplying only one of the, or selected, dXTPs within the limitations dictated by the nature of the sticky ends. After treatment with Klenow, the mixture is extracted with phenol/chloroform and ethanol precipitated. Treatment under appropriate conditions with S1 nuclease results in hydrolysis of any single-stranded portion.

Synthetic oligonucleotides are prepared by the triester method of Matteucci, et al. (J. Am. Chem. Soc., 103:3185 (1981)) or using commercially available automated oligonucleotide synthesizers. Kinasing of single strands for labeling is achieved using an excess, e.g., approximately 10 units of polynucleotide kinase to 10 pmole substrate in the presence of 50 mM Tris, pH 7.6, 10 mM $MgCl_2$, 5 mM dithiothreitol, 40 pmoles λ-32P-ATP (3000 Ci/mmole), 0.1 mM spermidine, 0.1 mM EDTA.

Ligations are performed in 15-30 µl volumes under the following standard conditions and temperatures: 20 mM Tris-Cl pH 7.5, 10 mM $MgCl_2$, 10 mM DTT, 33 µg/ml BSA, 10 mM-50 mM NaCl, and either 40 µM ATP, 0.01-0.02 (Weiss) units T4 DNA ligase at 0° C (for "sticky end" ligation) or 1 mM ATP, 0.3-0.6 (Weiss) units T4 DNA ligase at 14° C (for "blunt end" ligation). Intermolecular "sticky end" ligations are usually performed at 33-100 µg/ml total DNA concentrations (5-100 nM total end concentration). Intermolecular "blunt end' ligations (usually employing a 10-30 fold molar excess of linkers) are performed at 1 µM total ends concentration.

In vector construction employing "vector fragments", the vector fragment is commonly treated with bacterial alkaline phosphatase (BAP) in order to remove the 5′ phosphate and prevent religation of the vector. BAP digestions are conducted at pH 8 in approximately 150 mM Tris, in the presence of $Na^+$ and $Mg^{+2}$ using about 1 unit of BAP per µg of vector at 60° C for about one hour. Vector fragments subjected to this treatment are referred to herein as "BAPped". If unkinased oligodeoxyribonucleotides are used however, the vector fragments are not "BAPped". In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

Modification of DNA Sequences

For portions of vectors derived from cDNA or genomic DNA which require sequence modifications, site specific primer directed mutagenesis is used. This is conducted using a synthetic oligonucleotide primer complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered. Details of site specific mutation procedures are described below in specific examples.

Verification of Construction

In the constructions set forth below, correct ligations for plasmid construction are confirmed by first transforming E. coli strain MM294 obtained from E. coli Genetic Stock Center, CGSC 6135, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al., Proc. Natl. Acad. Sci. (USA), 62:1159 (1969), optionally following chloramphenicol amplification (Clewell, D. B., J. Bacteriol., 110:667 (1972)). The isolated DNA is analyzed by restriction enzyme mapping and/or sequenced by the dideoxy method of Sanger, F., et al., Proc. Natl. Acad. Sci. (USA), 74:5463 (1977) as further described by Messing, et al., Nucleic Acids Res., 9:309 (1981), or by the method of Maxam, et al., Methods in Enzymology, 65:499 (1980).

Transfer Vector Construction

Because the genome of AcNPV is so large (125kb), there are too many restriction sites to allow easy site-specific insertion of heterologous genes. Therefore, recombinant virus, containing the gene to be expressed, are derived through recombination between viral DNA and genetically engineered chimeric plasmids called transfer vectors.

The transfer vectors which have been described by Smith, et al., (1983 supra) were originally constructed by cloning the AcNPV EcoRI-1 fragment containing the polyhedrin gene into the EcoRI site of E. coli plasmid pUC8 (Vieira, J., et al., Gene, 19:259-268 (1982)). A series of plasmids or transfer vectors having single BamHI cloning sites at various locations in the polyhedrin gene were created as described (Smith et al., 1983 supra).

One of these, pAc373, has a single BamHI site 50 bp downstream from the polyhedrin cap site i.e., 8 bp before the polyhedrin ATG translation initiation codon (Smith et al., (1985) supra. The transfer vectors, pAc610 and pAc611 have the polylinker from M13mp10 and M13mp11, respectively, inserted at this BamHI site of pAc373 (Summers, J.D. et al., personal communication). Partial nucleotide sequence of pAc401 and pAc436 transfer vectors for the production of polyhedrin/foreign gene fusion proteins is also reported (Summers, et al., 1987, supra).

A. Preparation and Isolation of Recombinant AcNPV Virus

Detailed methods for the generation of recombinant virus can be found in European Patent Application No. 0127839 to G.E. Smith and M.D. Summers of the Texas A & M University System. In general, 2 µg of genetically engineered transfer vector DNA and 1 µg of AcNPV viral DNA are cotransfected onto monolayer culture cells of Spodoptera frugiperda. The infected cells usually show viral occlusions by day 3 or 4, with 10-90% of the cells being infected. The virus titer of the medium is expected to be $10^7$ pfu/ml and 0.1%-0.5% are expected to be recombinant virus.

Several methods for the detection of recombinant virus are known in the art. Visual detection of the plaques is best achieved using a low power dissecting microscope and observing the plaques on inverted plates with a black background and illumination from the side. More unequivocal methods for detecting recombinants are plaque hybridization using DNA probes to the cloned gene. Antibody probes to the product of the cloned gene may also be employed.

Isolation of the recombinant virus is achieved through plaque purification of serially infected monolayer cells overlayed with soft agar. After two or three cycles the recombinant virus would be seen as separate plaques showing the characteristic occlusion-negative morphology. The plaques, containing about 10,000 pfu of virus, are picked using a sterile Pasture pipet and transferred to 2 ml of medium.

Hosts Exemplified

Host strains used in cloning and expression herein are as follows:

For cloning and sequencing, and for expression of construction under control of most bacterial promoters, E. coli strain MM294 (supra), Talmadge, K., et al., Gene, 12:235 (L1980); Messelson, M., et al., Nature, 217:1110 (1968), was used as the host. For expression under control of the $P_L$ N-RBS promoter, E. coli strain K12 MC1000 lambda lysogen, $N_7N_{53}cI857SusP_{80}$, ATCC 39531 (hereinafter sometimes referred to as MC1000-39513 λgDG95 or DG95) may be used as well as E. coli strain DG116 also an MM294 strain λ CI857, bio T76, del HI); the bio T76 substitution deletes early λ function (N att) and the del HI deletion removes λDN from cro through att (del cro- $Bio^+$ $n^-$). This strain is deposited in the assignees culture collection under accession number CMCC 2298.

For M13 phage recombinants, E. coli strains susceptible to phage infection, such as E. coli K12 strain DG98 are employed. The DG98 strains has been deposited with ATCC July 13, 1984 and has accession number 1965.

Mammalian expression is carried out in COS lines and in derivatives thereof. Expression may also be carried out with appropriate vectors in transformed insect cell line in culture, in particular cells of Spodoptera frugiperda. Appropriate cell lines include Sf9 and IPLB-SF21. See generally Miller, et al. "An Insect Baculovirus Host-Vector System for High Level Expression Foreign Genes;" Genetic Engineering Principles and Methods Vol. 8, Setlow and Hollaender eds., Plenum Press, New York 1986, 277-298 and European Patent Publication No. 127,839 published December 12, 1984.

In addition to bacteria, eukaryotic microbes, such as yeast, may also be used as hosts. Laboratory strains of Saccharomyces cerevisiae, Baker's yeast, are most used although a number of other strains are commonly available. Vectors employing the 2 micron origin of replication are available (Broach, J. R., Meth. Enz., 101:307 (1983)), and other plasmid vectors suitable for yeast expression are known (see, for example, Stinchcomb, et al., Nature, 282:39 (1979)), Tschempe, et al., Gene, 10:157 (1980) and Clarke, L., et al., Meth. Enz., 101:300 (1983). Control sequences for yeast vectors include promoters for the synthesis of glycolytic enzymes (Hess, et al., J. Adv. Enzyme Reg., 7:149; (1968); Holland, et al., Biochemistry, 17:4900 (1978)). Additional promoters known in the art include the promoter for 3-phospoglycerate kinase (Hitzman, et al., J. Biol. Chem., (1980) 255:2073), and those for other glycolytic enzymes, such as glyceraldehyde-3-phosphate dehydrogenase, hexosekinase, pyruvate decarboxylase, phosphofructokinase, glucose-6-phosphate isomerase, 3-phosphoglycerate mutase, pyruvate kinase, triosphosphate isomerase, phosphoglucose isomerase, and glucokinase. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, ibid).

It is also believed terminator sequences are desirable at the 3′ end of the coding sequences. Such terminators are found in the 3′ untranslated region following the coding sequences in yeast-derived genes. Many of the vectors illustrated contain control sequences derived from the enolase gene containing plasmid peno46 (Holland, M. J., et al., J. Biol. Chem., 256:1385 (1981)) or the LEU2 gene obtained from YEp13 (Broach, J., et al., Gene, 8:121 (1978)), however any vector containing a yeast compatible promoter, origin of replication and other control sequences is suitable.

It is also, of course, possible to express genes encoding polypeptides in eukaryotic host cell cultures derived from multicellular organisms as described further hereinbelow. See, generally for example, Tissue Culture, Academic Press, Cruz and Patterson, editors (1973). Useful host cell lines include murine myelomas NS1, VERO and HeLa cells, COS cells, and Chinese hamster ovary (CHO) cells. Expression vectors for such cells ordinarily include promoters and control sequences compatible with mammalian cells such as, for example, the commonly used early and late promoters from Simian Virus 40 (SV40) (Fiers, et al., Nature, 273:113 (1978)), or other viral promoters such as those derived from polyoma, Adenovirus 2, bovine papilloma virus, or avian sarcoma viruses, or immunoglobin promoters and heat shock promoters. General aspects of mammalian cell host system transformations have been described by Axel, U.S. Patent No. 4,399,216 issued August 16, 1983. It now appears, also that "enhancer" regions are important in optimizing expression; these are, generally, sequences found upstream of the promoter region. Origins of replication may be obtained, if needed, from viral sources. However, integration into the chromosome is a common mechanism for DNA replication in eukaryotes. Plant cells are also now available as hosts, and control sequences compatible with plant cells such as the nopaline synthase promoter and polyandenylation signal sequences (Depicker,, A., et al., J. Mol. Appl. Gen., 1:561 (1982)) are available.

B. Insect Cell Culture

Methods for insect cell cultures are well known in the art and detailed procedures for their cultivation can be found in Summers et al., (1987 supra) or in EPO 127,839 to Smith, G.E. et al. The insect expression host of the current invention, Spodoptera frugiperda (Sf-9), is well suited to the production of heterologous proteins because of its ability to grow in either monolayer or suspension culture.

As monolayer cultures, Spodoptera frugiperda cells will divide every 18-24 hours depending on the culture media. The cells do not require carbon dioxide to maintain the pH of the medium and they will grow well at temperatures between 25-30° C. Subculturing is done 2 or 3 times a week when the cells are confluent. Because insect cells are loosely adherent they are easily resuspended without the need of proteases.

Suspension culture conditions will vary depending on the medium and culture volume and should be determined empirically. Subculturing is required when the cell density reaches 2 x $10^6$ cells/ml by replacing 80% or more of the culture with an equal volume of fresh medium. With suspension cultures larger than 500 ml it becomes necessary to aerate by either bubbling or diffusion.

The invention will be better illustrated in relationship to the following examples which are intended by the inventors to be exemplary and non-limiting.

## Specific Examples

### EXAMPLE 1

### Obtaining a cDNA Sequence for Pro-Urokinase

A. Identification of Cell Line Producing Urokinase Transcripts

An oligodeoxyribonucleotide probe was synthesized and purified and had the following sequence: 5′-ACTTGATGAAGTTCATTCGT-3′.

This probe was constructed based on the published DNA sequence of urokinase, Ny et al., Proc. Natl. Acad. Sci. USA, 81:5355-5359 (1984) and Verde et al., Proc. Natl. Acad. Sci. USA, 81:4727-4731 (1984). The oligodeoxyribonucleotide was labeled with $\gamma^{32}$P-ATP using DNA polynucleotide kinase and used to screen a variety of cell lines for mRNA transcripts encoding urokinase. All lines were screened as follows: Cells were grown in DMEM supplemented with 10% FCS at 37° C in roller bottles until they reached confluency. The RNA was prepared as described in Chirgwin et al. Briefly, the cells were lysed in 5 M guanidine isothiocyanate followed by centrifugation through a 5.7 M cesium chloride (CsCl) cushion; Poly $A^+$ RNA was prepared from the lysed cells by one selection cycle on oligo (dT) cellulose as described in Maniatis et al., supra.

In more detail, cells were lysed in a solution which contained 5 M guanidine isothiocyanate, 0.025 M Na-citrate, pH 7, 0.5% sarcosine and 8% 2-mercaptoethanol. Molecular biology grade CsCl was made up to 5.7 M or 40% w/v and buffered with 0.02 M Tris pH 7.5 and 0.002 M Na-EDTA. All solutions were prepared under RNase free conditions and passed through 0.45 µm Millipore filters before use. The lysed cells were then layered onto CsCl in SW28 ultracentrifuge tubes which contained layers of 10 ml 5.7 M CsCl followed with 6 ml 40% CsCl.

After centrifugation at 26,000 rpm for 18 hours, the RNA pellet was dissolved in $dH_2O$ and ethanol precipitated twice. Polyadenylated (Poly $A^+$) RNA was obtained by chromatography of the total RNA on oligo(dT) cellulose as described in Maniatis, supra (at page 197).

The RNA is dissolved in sterile $H_2O$ and heated to 65° C for five minutes. An equal volume of a solution containing 0.040 M Tris-Cl pH 7.6 1.0 M NaCl, 0.002 M EDTA and 0.2% SDS is quickly added and the sample is cooled to room temperature. The sample is then loaded on an oligo (dT) column that has been equilibrated with a buffer containing 0.020 M Tris pH 7.6, 0.5 M NaCl, 0.001 M EDTA and 0.1% SDS. The flow through is collected, heated to 65° C, cooled to room temperature and passed over the column once more. The column is then washed with 10 volumes of wash buffer (0.02 M Tris, pH 7.6, 0.1 M NaCl, 0.001 M EDTA, 0.1% SDS). Poly $A^+$ mRNA is eluted with aliquots of 0.01 M Tris pH 7.5, 0.001 M EDTA and ethanol-precipitated twice.

Northern blots were prepared by the electrophoresis of 10 µg of Poly $A^+$ RNA per lane through 1% agarose gels containing 0.5 M formaldehyde followed by blotting onto nitrocellulose filters. After baking the filters for 1.5 hours at 80° C, they were prehybridized (5 x SSC, 10x Denhardt's solution (0.2% polyvinylpyrrolidone, 0.2% Ficoll, 0.2% BSA), 0.1% SDS, 50 mM sodium phosphate pH 7.0, and 100 μg/ml tRNA) for one hour at 55° C. The blots were hybridized for 16 hours at 55° C in a similar solution that also contained 10% dextran sulfate and $10^6$ cpm per ml of oligodeoxyribonucleotide mentioned above labeled with $\gamma^{32}P$-ATP and polynucleotide kinase. The blot, shown in Figure 9, was washed at 55°C in 3 x SSC, 0.1% SDS. The cell line LD-1 was determined to have high levels of urokinase mRNA transcripts by this assay.

B. Preparation of a cDNA Library

The total Poly A+ RNA fraction isolated from the above-mentioned cell line was cloned as a cDNA library in a λgt10 vector essentially as described by Haynh et al., "Constructing and Screening cDNA Libraries in λgt10 and λgt11" in DNA Cloning, Vol. 1, IRL Press Ltd., Oxford, England, 1st ed. 1985, D. Miglover ed. In brief, single stranded cDNA is made using avian myeloblastosis virus (AMO) reverse transcriptase from the Poly $A^+$ mRNA fraction. The DNA RNA hybrids were denatured by heating. The denatured single-stranded DNA was made double stranded (ds) with DNA polymerase I using the single cDNA strands as self primers. Hairpin structures were removed with S1 nuclease. The dsDNA was size fractionated by preparative gel electrophoresis. The dsDNA was treated with EcoRI methylase to prevent digestion by EcoRI endonuclease and was blunt ended with DNA polymerase I large fragment (Klenow). Following extraction and purification, the blunt ended dsDNA is blunt end ligated to ([$\gamma^{32}P$]-labeled) EcoRI linkers. The linkers were cleaved with EcoRI to generate cohesive EcoRI ends, and the cDNA was purified on low melting point agarose. dsDNA with EcoRI cohesive ends were ligated to EcoRI digested λgt10 vectors. The library was then packaged in vitro and plated onto BNN102. The plaques were transferred to nitrocellulose filters and were probed using the above-mentioned $\gamma^{32}P$-labeled oligodeoxyribonucleotide. Probe positive plaques were expanded in strain BNN102 and the phage DNA was extracted and digested with HindIII and StuI.

The HindIII-StuI digest was ligated into plasmid pJV104, a derivative of pUC18 which contains the origin of replication of phage M13. The phage M13 origin of replication was originally obtained as a RsaI fragment from M13. The RsaI fragment was blunt end ligated to HindIII linkers and cloned for amplification into the HindIII site of a pBR322 derivative that contained a polylinker sequence. After amplification, the HindIII fragment containing the M13 origin of replication was isolated, repaired with Klenow, the large fragment of DNA polymerase I, and blunt-end ligated to NdeI linkers. After digestion with NdeI, the NdeI fragment containing the M13 origin of replication was ligated into NdeI-digested pUC18 under sticky end conditions to form pJV104.

After ligating the HindIII-StuI fragments from the λgt10 vector into pJV104, the ligation mixture was used to transform competent E. coli strain MM294. Colonies were grown up on agarose medium, lifted onto nitrocellulose filter papers and were lysed. DNA was fixed to the filter by treatment for five minutes with 0.5 mM NaOH, 1.5 M MaCl. Filters were then washed twice for five minutes each time with 1.5 M Tris pH 8, 3 M NaCl and and were air dried and baked at 80°C for two hours.

The filters for the screening were treated with prehybridization buffer and hybridized to the $\gamma^{32}P$ labeled probe as described above in Section A of the example, but both prehybridization and hybridization were carried out at 50° C. A plasmid, determined to be probe positive was designated JV104-17. The presence of the proper DNA fragment was confirmed by restriction fragment analysis and sequencing of selected fragments.

EXAMPLE 2

Construction of a DNA Sequence That Encodes the Amino-Terminal End of a Novel Plasminogen Activator

Four oligodeoxyribonucleotides were synthesized (A, B, C, and D in Figure 1 using an automated oligonucleotide synthesizer (Biosearch Model 8000, San Rafael, CA USA). Oligodeoxyribonucleotides A and B are complimentary and can hybridize to each other forming a segment of duplex DNA. This duplex has a four base single strand at one end that can hybridize to the single strand created by the digestion of DNA with the restriction enzyme Kpn1. At the other end of the duplex is a 9 base pair single strand.
Oligodeoxyribonucleotide C and D can anneal to each other forming a segment of duplex DNA. This duplex has a four base single strand at one end that can anneal to the single strand created by the digestion of DNA with the restriction enzyme HindIII. At the other end of the C-D duplex is a 9 base single strand that can anneal to the 9 base pair single strand at one end of the A-B duplex forming a single piece of duplex DNA containing oligodeoxyribonucleotides A, B, C, and D. This duplex was then inserted into a plasmid pCDB as described below and in Figure 2. The construction of plasmid pCDB is described in Example 5 herein.

The plasmid pCDB was digested with the enzyme HindIII and KpnI; the large (~2.6 kb) fragment was then isolated from a 0.5% agarose gel and mixed with oligodeoxyribonucleotides A, B, C, and D. After ligation of this mixture with T4 ligase, under sticky end conditions, competent E. coli cells (strain MM294) were transformed and plated on ampicillin containing agar plates. Resistant colonies were probed as described below. A colony that contained a recombinant plasmid (pJD12) consisting of the oligodeoxyribonucleotides A, B, C, and D inserted into the pCDB HindIII and KpnI sites was identified by colony hybridization using oligodeoxyribonucleotide B end labeled with $^{32}P$ using polynucleotide kinase. The plasmid pJD12 contains synthetic DNA that encodes a Met and gly residue, followed by the amino terminal end of a protein domain that is similar to the second kringle of t-PA.

The remainder of the kringle-like protein domain, a short linker region, and the protease domain of urokinase (up to the first EcoRI site in the region encoding the pro-urokinase protease domain) is encoded by the oligodeoxyribonucleotides E, F, G, and H shown in Figure 3. However, these oligodeoxyribonucleotides encode a gly residue rather than the lys residue corresponding to $lys_{158}$ in urokinase, i.e., a $lys_{158}$ to $gly_{158}$ mutein.

Oligodeoxyribonucleotides E, F, G, and H can form a single piece of duplex DNA in a manner analogous to that described for oligodeoxyribonucleotides A, B, C, and D above. This duplex has single stranded ends compatible with the ends generated by the enzyme HindIII. At the other end, the duplex has a single strand that is compatible with the ends generated by the enzyme SalI.

Oligodeoxyribonucleotides E, F, G, and H were ligated into pJD12 by digesting pJD12 with the enzymes SalI and HindIII and then isolating the large fragment from a 0.5% agarose gel. This fragment was mixed with oligonucleotides E, F, G, and H and the mixture ligated with T4 ligase under sticky end conditions. After ligation, a recombinant plasmid pJD14 as shown in Figure 3 was isolated by a method similar to that described for pJD12 except that oligonucleotide F, end labeled with $^{32}P$ using T4 poly nucleotide kinase, was used as the hybridization probe.

EXAMPLE 3

Construction of a Plasmid

Encoding a Novel Plasminogen Activator

The plasmid pJV104-17, which contains a partial cDNA clone of pro-urokinase, was digested to completion with HindIII and then partially digested with EcoRI. The ~2 kb EcoRI-HindIII fragment shown in Figure 5, was isolated from a low-melting point agarose gel. The plasmid pJD14 (described in Example 2 hereinabove) was digested with KpnI and partially digested with EcoRI. The approximately 300 bp KpnI-EcoRI fragment shown in Figure 5 was also isolated from a low melting point agarose gel. The plasmid pCDB, which contains convenient KpnI and HindIII sites, was digested with KpnI and HindIII and the large, approximately 2.6 kb KpnI-HindIII fragment was isolated from a low-melting point agarose gel as shown in Figure 5. The isolated fragments from the three plasmids were placed together in ligation buffer and ligated with T4 DNA ligase, under sticky end conditions. After ligation the mixture was used to transform competent E. coli strain MM294 and a colony containing recombinant plasmid pJD15 was isolated by separately preparing DNA from 12 ampicillin resistant colonies. The correct construction was verified by restriction fragment map analysis.

In order to place convenient restriction sites after the TGA translation termination codon, plasmid pJD15 was digested with HindIII and partially digested with BamHI. An approximately 4 kb fragment shown in Figure 6 was isolated from a low melting point agarose gel. Oligodeoxyribonucleotides K and L were synthesized and purified. After annealing, these oligomers generate a dsDNA having two single-stranded ends, one of which is complementary to the ends created by BamHI digestion and the other of which is complementary to the ends created by HinIII digestion. The 4 Kb fragment from pJD15 and oligonucleotides K and L were ligated using T4 ligase under sticky end conditions and used to transform competent E. coli strains MM294. Ampicillin resistant colonies were isolated and screened by colony hybridization using the $^{32}P$ labeled oligonucleotide L. A plasmid from a probe positive colony was designated pPD11 and had the expected restriction fragments resulting from the removal of the BamHI site from the coding region and the addition of a BamHI, EcoRV, and a KpnI site immediately after the TGA termination codon. Dideoxy sequencing across the small NcoI-NotI fragment of pPD11 revealed that two cytosine nucleotides in this fragment were absent. To correct the defect, pPD11 was digested with KpnI and EcoRV endonucleases. The approximately 1.1 Kb KpnI-EcoRV fragment was removed after electrophoresis of the digest on a low melting point agarose gel and was ligated, under blunt end conditions using T4 DNA ligase into a M13mp19 vector that had been previously digested with KpnI and HincII.

Single-stranded DNA from this recombinant phage was site specifically mutagenized using a oligodeoxyribonucleotide designated M, having the following sequence (the underlined region represents the added cytosine nucleotides): 5′-TGC CTC CTG CCT CCC GTG GAA TTC CAT-3′. Transformed plaques were identified by hybridization under strigent conditions to oligodeoxyribonucleotide M that had been labeled with $\gamma^{32}P$-ATP with T4 polynucleotide kinase. RF DNA from a probe positive plaque was sequenced across the KpnI-HindIII fragment to confirm the correct sequence. A plasmid carrying the correct sequence was designated pPD14. pPD14 was digested with NcoI and NotI restriction endonuclease. pPD11 was digested with the same endonucleases and BglI. The restriction fragments BglI-NcoI and NotI-BglI (both were 1695 bp from pPD11) were admixed in ligation buffer with the small fragment from pPD14 at a 1:10 molar excess and ligated using T4 DNA ligase under sticky end conditions. After ligation, the mixture was used to transform competent E. coli strain MM294. Colonies having the correct insert were identified by colony hybridization under strigent conditions using the oligodeoxyribonucleotide M labeled with $\gamma^{32}P$ as a probe. Probe positive colonies were grown up and the plasmids extracted. The correct construction was verified by restriction enzyme mapping and designated pPD17.

EXAMPLE 4

Construction of a Plasmid for the Expression of the Novel Plasminogen Activator in E. coli

To place the gene (PA-A) encoding a plasminogen activator according to the invention in an expression vector under $P_L$ promoter-gene N ribosome binding site transcriptional and translation control, the restriction enzyme sites NcoI and SphI and an ATG initiation codon were placed in plasmid pPLAPHβ.

Plasmid pLAPHβ is a plasmid having the $P_L$promoter and gene N ribosome binding site controlling the expression of the Aphβ gene. The Aphβ gene is contained in a HindIII-BamHI fragment. The plasmid further contains the positive retroregulatory element (PRE) 3′ to the Aphβ gene with a BamHI site directly 5′ of the PRE. Plasmid pLAPHβ was made from plasmid pFC54.t (ATCC number 39,739). Plasmid pLAPHβ is exactly the same as pFC54.t except that the latter plasmid has a HindIII-BamHI fragment encoding des-alanyl-IL-2 in place of the Aphβ encoding fragment. Thus, the following steps to place the expression of the plasminogen activator gene according to the invention under temperature sensitive $P_L$ promoter-gene N RSB and PRE control can also be carried out using pFC54.t.

To place the NcoI and SphI restriction endonuclease sites and ATG codon in the plasmid, two oligodeoxyribonucleotides, I and J shown in Figure 1, were synthesized and purified. The oligodeoxyribonucleotides I and J, hybridize to each other to form a piece of duplex DNA that has single strands on each end. One end of the duplex DNA is compatible with the single-stranded end created by digestion with BamHI restriction endonuclease and the other end is compatible with the single stranded end created by digestion with the restriction endonuclease HindIII. Plasmid pPLAPHβ was digested with BamHI and HindIII. The large fragment was isolated from low melting point agarose, mixed with oligodeoxyribonucleotides I and J, and ligated using T4 DNA ligase under sticky end conditions. E. coli strain DG95 was transformed with the ligation mixture and a colony harboring the recombinant plasmid designated pJD13, shown in Figure 4, was isolated by essentially the same method as that described for pJD12 except that oligonucleotide J, end-labeled with $^{32}P$ using polynucleotide kinase and $^{32}P$ ATP, was used.

The plasmid pJD13 contains the phage λ $P_L$promoter, the N gene ribosome binding site, and the positive retroregulatory element of the B. thuringiensis cry gene. This plasmid was digested with BamHI and NcoI and the large 5.2 kb BamHI-NcoI fragment was isolated from a low-melting point agarose gel. The plasminogen activator coding sequence was removed from pPD17 by digestion with NcoI and BamHI, followed by isolation of the ~1.1 kb NcoI-BamHI fragment from an agarose gel. The two isolated fragments were mixed and ligated with T4 ligase under sticky end conditions. This mixture was then used to transform competent E. coli strain DG116. A colony harboring the recombinant plasmid pPD7, Figure 7, was isolated and the plasmid verified by restriction analysis.

EXAMPLE 5

Construction of a Vector for the Expression of the Novel Plasminogen Activator in COS Cells

An expression vector for use in COS cells, plasmid pCDB was constructed and the DNA sequence (PA-A) encoding the novel plasminogen activator was inserted therein. The expression vector is the same as pCD-X, described in Okayama and Berg, Molecular and Cell Biol., 3(2):280-289 (1983), except that the cDNA sequence of pCD-X is replaced by a polylinker having a KpnI site at one end and a PstI site at the other. The expression vector pCDB may be made as follows. Plasmid pCDV1 (Okayama and Berg, supra) is digested with HindIII and KpnI endonucleases and the large fragment is isolated by electrophoresis in a low melting point agarose gel. Plasmid pL1 (Okayama & Berg, supra) is digested with HindIII and PstI endonucleases and the small fragment is also isolated by electrophoresis in a low melting point agarose gel. Two strands of a double-stranded oligodeoxyribonucleotide polylinker sequence are synthesized by conventional means on an automated DNA synthesizer. This double stranded DNA has one PstI cohesive end and one KpnI cohesive end and a number of convenient restriction sites in between the cohesive ends. The double stranded polylinker has the following sequence:

5'- GGAGCTCAGATCTTCTAGAGAATTCTCGAGCGGCCGCATCGATGGTAC-3'
3'-ACGTCCTCGAGTCTAGAAGATCTCTTAAGAGCTCGCCGGCGTAGCTAC-5'.

The two plasmid fragments and the double-stranded polylinker are ligated with T4 DNA ligase. After ligation, the mixture is used to transform competent E. coli strain MM294. The correct plasmid designated pCDB is verified by restriction fragment analysis.

To clone the DNA encoding the plasminogen activator according to the invention into the vector, plasmid pPD17 and plasmid pCDB were digested with KpnI. pCDB has a unique KpnI site and the plasminogen activator sequence in pPD17 is bordered by KpnI sites. the ~1 kb fragment of pPD17 and the linearized pCDB were mixed together under sticky end conditions with T4 DNA ligase. After ligation, the mixture was used to transform competent E. coli strain MM294. Transformed colonies were identified by ampicillin resistance on selection medium. The correct plasmid construction was verified by restriction analysis. One such colony was designated pPD8 and is shown in Figure 7.

EXAMPLE 6

Construction of New Baculovirus Transfer Vectors

A. Construction of pAcCl

pAcCl is similar to pAc401 (described previously in the section entitled "Transfer Vector Construction") except that the recognition site for EcoRI endonuclease has been removed. To accomplish this, pAc401 was digested to completion with EcoRI and the ends were made blunt using Klenow fragment. After ligation and transformation, candidates were screened for the absence of an EcoRI site.

B. Construction of pAcC2

pAcC2 is similar to pAc436 (described previously in the section entitled Transfer Vector Construction) except that the recognition site for EcoRI endonuclease has been removed. To accomplish this, pAc436 was digested to completion with EcoRI and the ends were made blunt using Klenow fragment. After ligation and transformation, candidates were screened for the absence of an EcoRI site.

C. Construction of pAcC3

pAcC3 differs from pAcC2 in that an NcoI restriction site has been introduced at the ATG translational start of the polyhedrin gene. To accomplish this the new transfer vector, pAcC2, was digested to completion with SmaI endonuclease. Following phenol extraction and ethanol precipitation, SmaI-digested pAcC2 was dissolved in TE buffer (10 mM Tris-HCl pH 7.4; 1 mM EDTA). In a 50 μl volume of ExoIII buffer (50 mM Tris-HCl pH 8.0; 5 mM $MgCl_2$; 10 mM β-mercaptoethanol), 10 μg of SmaI-digested pAcC2 was treated with 50 units of E. coli Exonuclease III (ExoIII) at 30° C for 5 minutes. The sample was phenol extracted and ethanol precipitated twice. Then 50 pmoles of a primer EK85, AACCTATAAACCATGGCGGCCCGG, was kinased with cold ATP in a 20 μl reaction volume (50 mM Tris-HCl pH 7.8; 10 mM $MgCl_2$; 10 μM βmercaptoethanol). To 5 mg of ExoIII-treated pAcC2 was added 10 pmoles of kinased ED85 in a final volume of 20 ml NET (100 mM NaCl; 10 mM Tris-HCl pH 7.5; 1 mM EDTA) buffer. To anneal the plasmid and primer, the reaction was heated to 65° C for 10 minutes, incubated at 37°C for 10 minutes and placed on ice. The extension reaction was performed by adding 20 μl 2x Klenow buffer (40 mM Tris-HCl pH 7.5; 20 mM $MgCl_2$; 2 mM β-mercaptoethanol) containing 1 μl 10 mM dNTPs, 1 μl 10 mMATP, 1 μl (about 2 units) Klenow fragment and 1 μl (about 1-2 units) T4 DNA ligase. The reaction was incubated at 16° C for about 4 hours and then transformed into MM294. Miniprep DNAs were screened by analyzing for the presence of an NcoI site. Miniprep DNA was then used to retransform and obtain the desired pure clone.

D. Construction of pAcC4 and pAcC5

pAcC4 and pAcC5 are derivatives of pAcC3 containing a polylinker sequence at the SmaI site. The polylinker contains recognition sites for restriction endonucleases SmaI, KpnI, PstI, BglII, XbaI (cleavable when DNA is unmethylated), EcoRI, BamHI, and BclI. pAcC4 contains the sequence in one orientation while pAcC5 contains the polylinker in the opposite orientation (see Figure 14). To construct these vectors pAcC3 was digested to completion with XmaI endonuclease and ligated with two complementary self-annealed oligomers having the sequence

5'-CCGGGTACCTGCAGATCTAGAATTCGGATCCTGATCA-3'

3'-CATGGACGTCTAGATCTTAAGCCTAGGACTAGTGGCC-5'

After transformation of MM294, miniprep DNA of transformants were analyzed for the presence of restriction sites in the polylinker sequence.

EXAMPLE 7

A. Construction of Recombinant Baculovirus Transfer Vectors Containing a Novel Plasminogen Activator

The source of the novel plasminogen activator (PA-A) gene to be inserted into the pAcC3 transfer vector was pPD17 described above. pAcC3 was digested with both NcoI and SmaI endonucleases. As previously mentioned, the NcoI site of pAcC3 is located at the translational start of the polyhedrin gene. pPD17 similarly contains an NcoI site at the translational start of PA-A and double digestion with EcoRV yields a fragment containing the PA-A gene. After ligation with T4 ligase, the desired transformant carries the recombinant transfer vector, PPD10, with the PA-A gene inserted at the regenerated translational start codon in pAcC3. There is no signal peptide sequence in pAcC3; also there is no signal peptide sequence to direct secretion of PA-A in pPD10.

The next step was to try to produce a secreted form of PA-A by placing signal sequences in front of the coding region. Two such signal sequences were used. One sequence was designed by choosing preferred, amino acid residues (for eukaryotic proteins) in positions -13 through +2 relative to the cleavage site between the signal sequence and the mature protein (Von Heijne, G., 1986, supra). The N-terminal portion of this leader (residues -14 to -21) is derived from the rabbit uteroglobin signal sequence (von Heigne, G., J. Mol. Biol., 173:243 (1984)). Complementary oligonucleotides encoding this consensus signal peptide and having the sequences:

```
    M   A   L   A   I   T   L   A   L   L   L   L   L   L   L   L   P   G   C
5'CATGGCGTTAGCTATTACTCTTGCATTATTGCTTCTCCTACTGTTATTGCCTGGGTGT
  3'   CGCAATCGATAATGAGAACGTAATAACGAAGAGGATGACAATAACGGACCCACA

  +1  +2
 W   A   Q   P   G
TGGGCTCAACCCGG 3'
ACCCGAGTTGGGCCGTAC 5'
```

were chemically synthesized. The NcoI sticky ends of this synthetic oligonucleotide facilitated ligation into NcoI-digested pPD10 using T4 ligase. These synthetic oligonucleotides were designed to regenerate only the NcoI site preceding the DNA sequence encoding the consensus signal peptide sequence. The desired construct was designated pPD18.

The second signal sequence used included the t-PA signal peptide sequence, possibly part of the t-PA prosequence (the cleavage site between the signal and prosequences is not known) and the 5 residues immediately N-terminal to the t-PA kringle-2. The ability of the DNA encoding these regions to cause the secretion of PA-A was tested because these regions had been reported to successfully induce the secretion of a t-PA molecule that lacked the finger, epidermal growth factor and kringle-1 domain. (Van Zonneveld et al., (1986) supra). Complementary oligonucleotides encoding this t-PA signal peptide and having the sequences:

```
    M   D   A   M   K   R   G   L   C   C   V   L   L   L   C   G   A   V   F   V
5' CATGGATGCAATGAAGAGAGGGCTCTGCTGTGTGCTGCTGCTGTGTGGAGCAGTCTTCGTT
3'     CTACGTTACTTCTCTCCCGAGACGACACACGACGACGACACACCTCGTCAGAAGCAA
S   P   S   H   E   G   N   S   D
TCGCCCAGCCATGAGGGAAACAGTGA 3'
AGCGGGTCGGTACTCCCTTTGTCACTGTAC 5'
```

were ligated using T4 ligase into NcoI-digested pPD10. Transformants were screened as for pPD18 and the desired construct was designated pPD19.

B. Expression of a Novel Plasminogen Activator in Insect Cells

Sf9 insect cells were cotransfected with pPD10 and wild type baculovirus. Analysis of supernatants and cells of this initial transfection on Western blots showed that the cells but not the supernatants contained PA-A protein. Western blot analysis of cell sonicates showed that cells contained a substantial amount of urokinase antigen with a lower molecular weight (30,000) than expected for PA-A (MW 39,000).

The biological activity of the intracellular PA-A was also determined. After extraction of Sf9 cells with 0.1% SDS or 0.5% NP10, the PA-A remained in the pellet (30 min at 30,000 rpm). The PA-A could be extracted from this pellet with 1.0% SDS; when this mixture was diluted to 0.1% SDS, the PA-A remained in the supernatant after a 10 min spin at 14,000 rpm. However, using the casein plate assay, no PA activity could be detected in this 0.1% SDS supernatant. Urokinase activity was not affected by an identical treatment with SDS. Since both PA-A and urokinase have almost identical protease domains, this suggested that PA-A produced in Sf9 cells might not be properly folded. Alternatively, the PA-A might be partially degraded as supported by the lower molecular weight of PA-A produced in insect cells observed on Western blots.

The intracellular level of PA-A was determined on days 2 through 6 post infection. The Sf9 cells contained peak levels of PA-A on day 4.

A Western blot showed that both pPD18 and pPD19 resulted in the secretion of PA-A into the Sf9 cell medium. PA-A from both constructs appeared as a closely spaced doublet on the Western blot. These doublets may be due to differential glycosylation as the PA-A molecule contains three potential N-glycosylation sites. The molecules secreted by the t-PA signal peptide are slightly larger than those produced by the consensus signal peptide, presumably because this construct includes extra residues from t-PA after the leader peptidase cleavage site. Both the consensus and t-PA leader resulted in the secretion of active PA-A.

The PA assays used involve the conversion of plasminogen to the active protease plasmin by PA. The amount of plasmin produced is then measured by either the development of a clear zone on a casein-agarose plate or by the cleavage of a chromogenic peptide substrate. Both of these assays indicate that pPD18 and pPD19 direct the production of about 20 units of PA per ml. The units are determined by comparison to a urokinase international-unit standard.

EXAMPLE 8

Plasmid (pJD22) and Gene (cPA-P) Encoding a PlasminogenActivator Comprising Plasminogen kringle-1,a Proline Rich Linker, and the Pro-Urokinase Protease Domain

The plasmid pPD18 was digested with NcoI and NotI; the resulting fragments were separated by electrophoresis through low melting point agarose and the large fragment encoding most of a urokinase-like protease domain, was isolated. This fragment was ligated to the oligonucleotides JD90, JD91, JD92, and JD93 described in Table 1 below. These four oligonucleotides anneal together to form a double-stranded segment of DNA with a single-strand extension at both ends; one of these ends is compatible with the single strand created by the enzyme NcoI and the other end is compatible with the single strand generated by the enzyme NotI. After ligation, the mixture was heated to 65° C for five minutes, diluted from 20 µl to 200 µl with 10 mM Tris pH 7.5 EDTA 1 mM, cooled to room temperature and used to transform competent E. coli (strain MM294) cells. A colony containing pPD20 was identified by restriction mapping.

pPD20 was digested with XmaI and NotI. The large fragment was isolated from low melting point agarose after electrophoresis. This segment was ligated to oligonucleotides JD94, JD95, JD96, and JD97 described in Table 1 below. These four oligonucleotides anneal to form a double-stranded segment of DNA with single-stranded XmaI and NotI extensions. After ligation, E. coli strain MM294 were transformed and a colony containing pPD21 was isolated by hybridization with JD96. The oligonucleotide-derived DNA in pPD21 was sequenced and found to contain two incorrect bases. The errors were in the region encoding the signal sequence (corrected with oligonucleotide JD123) and in kringle-1 or plasminogen (corrected with oligonucleotide JD124).

These errors were corrected by in vitro mutagenesis. This was done by placing the small EcoRV-EcoRI fragment of pPD21 in the vector M13mp19 that had been digested with HincII and EcoRI, thereby creating mpJD18. Single-stranded mpJD18 was prepared and used a template for the mutagenic oligonucleotide JD123. A phage with the correct sequence was isolated by hybridization with JD123 and termed mpJD18-123; single-stranded DNA was prepared from this phage and used as a template for the mutagenic oligonucleotide JD124. A phage containing the corrections made by both JD123 and JD124 was then identified by hybridization with JD124 and designated mpJD18-123/124. The NotI-NcoI fragment of mpJD18-123/124 that encodes the plasminogen Kingle 1 was isolated from low melting point agarose after electrophoresis. This fragment was ligated to the large NcoI-NotI fragment of pPD18 that had been isolated from low melting point agarose after electrophoresis. Competent E. coli (strain MM294) cells were transformed with this ligation mixture and pJD22 was identified by restriction mapping.

Similarly, plasmid pLP19 was constructed which encodes cPA-P2, a gene identical to that encoding cPA-P except that the codon corresponding to $lys_{158}$ in urokinase encodes a lys in cPA-P2 and a gly in cPA-P. To construct pLP19, the oligonucleotide JD89B, illustrated in Table 1, is used for site-specific mutagenesis to change gly → lys at the position corresponding to $lys_{158}$ urokinase of the gene encoding cPA-P in plasmid pJD22.

Table 1

| Oligonucleotide Number | Sequence |
|---|---|
| JD89B | GCCGCGCTTTAAGATTATTGGGGG |
| JD90 | CATGGCGTTAGCTATTACTCTTGCATTATTGCTTCTCCTACTGTTATTGCCTGGGTGTTGGGCTAGCGAATGCAAAACCGGTGACGGTA |
| JD91 | AAAACTACCGTGGTACCATGTCCAAAACCAAAAACGGTATCACCTGCCAGAAATGGTCCTCCACCTCCCCGCACCGTCCGCGTTTCTCCCCGGGATGGCGC |
| JD92 | GGCCGCGCCATCCCGGGGAGAAACGCGGACGGTGCGGGGAGGTGGAGGACCATTTCTGGCAGGTGATACCGTTTTTGGTTTTGGACATGGTACC |
| JD93 | ACGGTAGTTTTTACCGTCACCGGTTTTGCATTCGCTAGCCCAACACCCAGGCAATAACAGTAGGAGAAGCAATAATGCAAGAGTAATAGCTAACGC |
| JD94 | CCGGCTACCCACCCGTCCGAAGGTCTGGAAGAAAACTACTGCCGTAACCCGGACAACGACCCGCAGGGTCCGTGGTGCTACACCACCGACCCGGAA |
| JD95 | AAACGTTACGACTACTGCGACATCCTCGAGTGCGAAGAAGAACCGTCCTCCCCGCCGGAAGAACTGAAATTCCAGTGCGGTCAGAAAACCCTGC |
| JD96 | GGCCGCAGGGTTTTCTGACCGCACTGGAATTTCAGTTCTTCCGGCGGGGAGGACGGTTCTTCTTCGCACTCGAGGATGTCGCAGTAG |
| JD97 | TCGTAACGTTTTTCCGGGTCGGTGGTGTAGCACCACGGACCCTGCGGGTCGTTGTCCGGGTTACGGCAGTAGTTTTCTTCCAGACCTTCGGACGGGTGGGTAG |
| JD102 | TCGAGTGCTCCGGCTCTGGTCCGCCACCCCCTCCGCCGTCTGGTTCCGGCCCTCCCCCGCCACCGCCATCCGGTTCTGGTTGCGGTCAGAAAACCCTGC |
| JD102B | TCGAGTGCTCCGGCTCTGGTCCGCCACCCCCTCCGCCGTCTGGTTCCGGCCCTCCCCCGCCACCGCCATCCGGTTCTGGTTGCGGTCAGAAAACCCTGC |
| JD103 | GGCCGCAGGGTTTTCTGACCGCAACCAGAACCGGATGGCGGTGGCGGGGGAGGGCCGGAACCAGACGGCGGAGGGGGTGGCGGACCAGAGCCGGAGCAC |
| JD103B | GGCCGCAGGGTTTTCTGACCGCAACCAGAACCGGATGGCGGTGGCGGGGGAGGGCCGGAACCAGACGGCGGAGGGGGTGGCGGACCAGAGCCGGAGCAC |
| PD18 | CATGGCGTTAGCTATTACTCTTGCATTATTGCTTCTCCTACTGTTATTGCCTGGGTGTTGGGCTCAACCCGG |
| PD19 | CATGCCGGGTTGAGCCCAACACCCAGGCAATAACAGTAGGAGAAGCAATAATGCAAGAGTAATAGCTAACGC |
| PD24 | CCCGTCCTGCGCTGACGGTGGCGGACCGTCCTCTCCGCCAGAAGAGCTGAAATTCCAGTGCGGTCAGAAAACCCTGC |

EXAMPLE 9

Plasmid (pPD26) and Gene (cPA-P1) Encoding a Plasminogen Activator Comprising Plasminogen kringle-1, a Hybrid Linker, and the Pro-Urokinase Protease Domain

Plasmid pPD18 was digested with XhoI and NotI, the large fragment was isolated from low melting point agarose after gel electrophoresis and ligated to JD102 and JD103 (two complementary oligonucleotides illustrated in Table 1, that form XhoI and NotI compatible single-stranded ends). Competent MM294 were transformed with the ligation mixture and a colony harboring pPD28 was identified by restriction mapping mini-prepped DNA from several colonies. pPD26 was constructed by a three-way ligation of the following fragments: the small XhoI-BamHI fragment of pPD28 (encoding linker and protease domain), the large NcoI-BamHI fragment of pPD18 (a baculovirus transfer vector), and the small NcoI-XhoI fragment of pJD22 (encoding the signal sequence and plasminogen kringle-1). All three of these fragments were isolated from low melting point agarose after electrophoresis. After ligation the plasmids were transformed into E. coli as described for the construction of pPD20. A colony harboring pPD26 was identified by restriction mapping DNA from several colonies.

EXAMPLE 10

Plasmid (pJD25) and Gene (cPA-A1) Encoding a Plasminogen Activator Comprising t-PA Kingle 2, a Proline Rich Linker, and the Pro-Urokinase Protease Domain

Plasmid pPD23 was constructed by ligating JD100 and JD101 (complementary oligonucleotides, illustrated in Table 1, that generate AatII and NotI compatible single-stranded ends) to the large NctI-AatII fragment of pPD8α (encoding part of the protease domain) that had been isolated from low melting point agarose after electrophoresis. A colony that contained pPD23 was identified by restriction mapping plasmids from several colonies.

pJD19 was constructed by ligating the following three fragments: the 900 bp AatII-BamHI fragment of pPD23, the large HindIII-BamHI fragment of pCBD, and the 700 bp HindIII-AatII fragment of pPD8. These fragments were isolated from low melting point agarose after electrophoresis. After ligation, competent E. coli cells (MM294) were transformed and a colony containing pJD19 was selected after restriction mapping plasmids from several colonies.

pJD21 was constructed by ligating the small NcoI-BamHI fragment of pJD19 to the large NcoI-BamHI fragment of pPD21. These fragments were isolated from low melting point agarose after gel electrophoresis. After transformation of E. coli, a colony containing pJD21 was identified after restriction mapping DNA from several colonies.

pJD25 was constructed by digesting pJD21 with NcoI, isolating the linearized plasmid from low melting point agarose, following electrophoresis, ligating this fragment with PD18 and PD19 (two complementary oligonucleotides, illustrated in Table 1, that form NcoI compatible single-stranded ends, and transforming E. coli. A colony that contained pJD25 was identified by restriction mapping plasmids from several colonies.

EXAMPLE 11

Plasmid pPD30 and Gene (cPA-A2) Encoding Plasminogen Activator Comprising t-PA kringle-2, a Hybrid Linker, and the Pro-Urokinase Protease Domain

The large AatII-NotI fragment of pPD8 was isolated from low melting point agarose after electrophoresis and ligated to PD23 and PD24 (two complementary oligonucleotides, illustrated in Table 1, that form AatII and NotI compatible single-stranded units). E. coli strain MM294 were transformed with this mixture as described for the construction of pPD20. A colony containing pPD29 was identified by restriction mapping plasmids from several colonies.

pPD30 was constructed by ligating the small NcoI-AatII fragment of pPD18, the large NcoI-BamHI fragment of pPD18, and the small AatII-BamHI fragment of pPD29. These fragments were isolated from low melting point agarose after electrophoresis. A colony containing pPD30 was identified by hybridization with oligonucleotide pPD23.

EXAMPLE 12

Construction of a Plasmid (pLP6) Encoding cPA-A3

The coding region of cPA-A3 is the same as cPA-A2 except that the codon corresponding to that for $lys_{158}$ in urokinase encodes a lys in cPA-A3 and a gly in cPA-A2. To construct the coding region for pLP6, the small KpnI-BamHI fragment of pJD22, comprising the urokinase protease domain, and the large KpnI-BamHI fragment of M13mp19 were isolated from low melting point agarose and ligated together. This mixture was used to transform E. coli (strain DG98) and single-stranded DNA was prepared from one of the phage plaques (mpLP2). The oligonucleotide JD89B, illustrated in Table 1, was then used to convert the gly codon (at the position corresponding to the codon for $lys_{158}$ in urokinase) to a lys codon. RF DNA of this new phage (mpLP3) was prepared, digested with BamHI and NotI, and the small fragment comprising the protease domain coding sequence was isolated from low melting point agarose. The large NotI-BamHI fragment of pPD30 was also isolated. These two isolated fragments were then ligated together, used to transform MM294 cells and a colony containing pLP6 was isolated by hybridizing a Southern blot containing DNA from several colonies with the $^{32}P$ labeled oligonucleotide JD89B.

EXAMPLE 13

Construction of Plasmids pLP8, pLP14, and pLP18, Encoding cPA-A4

The region encoding cPA-A4 is identical to that encoding cPA-A except that the codon corresponding to $lys_{158}$ in urokinase encodes a lys in cPA-A4 and a gly in cPA-A. To construct pLP8, the small NotI-BamHI fragment of pLP6 and the large NotI-BamHI fragment of pPD18 were isolated from low melting point agarose, ligated together, and used to transform MM294 cells. DNA from several colonies was digested with NotI and BamHI, blotted onto nitrocellulose paper, and hybridized with HD89B (see Table 1) to identify a colony containing pLP8.

The genes encoding cPA-A5 and cPA-A6 were also constructed. The regions encoding cPA-A5 and cPA-A6 are identical to that encoding cPA-A4 except that codons for certain asparagine residues have been changed to codons for glutamine, thereby removing potential N-glycosylation sites.

Plasmid pLP14 encodes cPA-A5, which contains an asn→gln substitution at the residue corresponding to $asn_{302}$ in the urokinase protease domain. The specific gene sequence can be made using site-specific mutagenesis with oligonucleotide LP11 (illustrated in Table 2). Similarly, asn→gln substitutions were introduced at residues 184 and 218, numbered in accordance with the t-PA sequence, located within the kringle-2 domain to construct cPA-A6. Site-specific mutagenesis can be performed using oligonucleotides LP12 and LP13 respectively, (illustrated in Table 2), to modify residues 184 and 218. A plasmid encoding cPA-A6, termed pLP18, was constructed.

## Table 2

| Oligonucleotide Number | Sequence |
|---|---|
| JD107 | CATGGATGCAATGAAACGAGGACTATGCTGCGTTCTGCTGCTGTGC GGTGCTGTTTTCGTTTCTCCGTCCCAGGAAATCCAC |
| JD108 | GCTCGTTTCCGTCGTGGTGCTCGTTCCTACCAGGTTATCTGCCGTG ACGAAAAAACCCAGATGATCTACCAGCAGCACCAGAGCT CGATGGATCGAACG |
| JD111 | CTAGCGTTCGATCCATCGAGCTCTGGTGCTGCTGGTAGATCATCTG GGTTTTTTCGTCACGGCAGATAACCTGGTAGGAACGAGCACCACG |
| JD112 | ACGGAAACGAGCGTGGATTTCCTGGGACGGAGAAACGAAAACAGCA CCGCACAGCAGCAGAACGCAGCATAGTCCTCGTTTCATTGCATC |
| JD126B | CAGTGCCACTCCGTTCCGGTTAAATCCTCACCACCTGAAGAGCTGA AATTCCAGTGCGGTCAGAAAACCCTGC |
| JD127B | GGCCGCAGGGTTTTCTGACCGCACTGGAATTTCAGTTCTTCCGGCG GGGAGGATTTAACCGGAACGGAGTGGCACTGAGCT |
| JD133 | GCCTACCGTGGTACCCACAGCCTCACC |

### EXAMPLE 14

Construction of a COS Cell Expression Plasmid (pLP15) Encoding cPA-A3

First, the large HinIII-BamHI fragment of pCDB was isolated. Second, pCDB was digested with EcoRI, the single-stranded ends filled in with Klenow and dNTPs, and then digested with HindIII. The small fragment containing, the SV40 promoter was isolated from a low melting point agarose gel. Third, pLP6 was digested with NcoI, the single-stranded ends were filled in with Klenow and dNTPs, digested with BamHI, and then the small NcoI-BamHI fragment was isolated from low melting point agarose. All three isolated fragments were then ligated together and the mixture was used to transform MM294 cells. Restriction mapping of DNA from several colonies confirmed the construction of pLP15.

### EXAMPLE 15

Construction of a COS Cell Expression Plasmid pLP16 Encoding cPA-P2

The region encoding cPA-P2 is identical to the region encoding cPA-P except that the codon corresponding to $lys_{158}$ in urokinase is a lys codon in the cPA-P2 coding region and a gly codon in the cPA-P coding region. The plasmid pLP15 was digested with XbaI, the single-stranded ends filled in with Klenow and dNTPs, digested with NotI, and the large fragment isolated from low melting point agarose. The plasmid pJD22 was digested with NcoI, the single-stranded ends filled in with Klenow and dNTPs, digested with NotI, and the small NcoI-NotI fragment was isolated from low melting point agarose. These two isolated fragments were ligated with the mixture used to transform MM294 cells. A colony harboring pLP16 was identified by restriction mapping DNA from several colonies.

### EXAMPLE 16

Plasmid (pLA7) and Gene (fPA-FP) Encoding a Plasminogen Activator Comprising t-PA finger, Plasminogen kringle-1, and the Urokinase Protease Domain

The large NcoI-NheI fragment of pJD22, which encodes the plasminogen kringle-1 and the urokinase protease domain, is isolated and ligated to the oligonucleotides JD107, JD108, JD111, and JD112, which upon assembly, encode the N-terminal portion of the finger domain of t-PA. These four oligonucleotides are illustrated in Table 2 above. After the resultant plasmid is isolated from E. coli transformed with the above ligation mixture, the large SacI-NheI fragment is isolated. This fragment (encoding the amino terminal lend of the finger domain, kringle-1 of plasminogen, and the urokinase protease domain) is then ligated to the oligonucleotides JD109 and JD120, illustrated in Table 2, which encode the carboxy terminal portion of the t-PA finger domain and a linker sequence. The resultant plasmid encodes fPA-FP. A plasmid encoding fPA-FP was designated pLP7a.

fPA-FP1 is identical to fPA-FP except that the residue corresponding to $lys_{158}$ of urokinase is a gly residue in fPA-FP and a lys residue in fPA-FP1. A plasmid encoding fPA-FP1 was made by replacing the NotI-BamHI fragment of pLP7a, which encodes the protease domain, with the NotI-BamHI fragment of pLP6, which encodes the protease domain. This new plasmid was termed pLP21.

EXAMPLE 17

Plasmids (pLP4a and pLP20) and Genes (fPA-F and fPA-F1) Encoding Plasminogen Activators Comprising t-PA Finger Domain and the Urokinase Protease Domain

The large SacI-NotI fragment of pLP7a is isolated and ligated to the oligonucleotides JD126B and JD127B, illustrated in Table 2. The resultant plasmid encodes fPA-F and was designated pLP4a.

fPA-F is identical to fPA-F1 except that the residue corresponding to $lys_{158}$ of urokinase is a gly residue of fPA-F and a lys residue in fPA-F1. A plasmid encoding fPA-F1 was constructed by replacing the small NotI-BamHI fragment of pLP4a, which encodes the protease domain, with the small NotI-BamHI fragment of pLP6, which encodes the protease domain. This new plasmid was called pLP20.

EXAMPLE 18

Plasmid and Gene (fPA-FA) Encoding a Plasminogen Activator Comprising t-PA Finger Domain, t-PA kringle-2, and the Urokinase Protease Domain

A plasmid encoding fPA-FA (the t-PA finger domain, the t-PA kringle-2, and the urokinase protease domain) is constructed as follows. A KpnI site is created by site directed mutagenesis using oligonucleotide JD133 (illustrated in Table 2) in the region encoding the t-PA kringle-2 using either pPD18 or pPD30 as the starting material. The large NcoI-KpnI fragment is isolated from the individual starting plasmid and ligated to the isolated small NcoI-SacI fragment of pLP7a in a four piece ligation with oligonucleotides JD134 and JD135 (illustrated in Table 2). The resultant plasmid encodes fPA-FA.

EXAMPLE 19

Fibrin Stimulation of Hybrid Plasminogen Activators

In order to demonstrate that the kringle- and finger domains of the hybrid plasminogen activators were capable of interacting with fibrin, a fibrin stimulation assay was used wherein the change in PA activity in response to the presence of fibrin is performed as follows. A known amount of hybrid PA, urokinase or t-PA is assayed in the presence or absence of Desafib (a solution of soluble fibrin fragments available from American Diagnostica). The final concentration of Desafib, if added, is 0.04 µg/ml. To quantitate the amount of fibrin stimulation, a chromogenic assay for plasminogen activation is employed using glu-plasminogen and spec pL, a chromophore (available from American Diagnostica). The fibrin stimulation of the PAs following exposure to fibrin is calculated by:

$$\frac{(A_{405}\ \text{presence of fibrin}) - \text{background}}{(A_{405}\ \text{absence of fibrin}) - \text{background}} \times 100$$

The results from two separate experiments, in Table 3, show that urokinase or scu-PA are stimulated by fibrin less than twofold, while the certain hybrid PA molecules show varying degrees of fibrin stimulation ranging from twofold to fivefold.

Table 3

| Compound | Fibrin Stimulation | |
|---|---|---|
| UK | 1.7 | 1.4 |
| scu-PA | 1.9 | 0.94 |
| PA-A2(pPD30) | 5.5 | 5.5 |
| PA-A3(pLP6) | 3.7 | 2.3 |
| PA-P(pJD22) | 2.1 | - |

## EXAMPLE 20

### Saturation Mutagenesis Assay

The following example describes the saturation mutagenesis of the DNA encoding the asp residue in the protease domain that corresponds to $asp_{355}$ of urokinase. The numbering of the residues identified in this example follows urokinase numbering.

First, in vitro mutagenesis is used to convert the codons encoding $ala_{342}ala_{343}$ in pJD22 to GCC,GCG, thereby creating a SacII site. Second, in vitro mutagenesis is used to convert the codons encoding $leu_{364}gln_{365}$ to CTG,CAG, thereby creating a PstI site. The resulting baculovirus transfer vector with the unique new PstI and SacII sites is termed pJD26. pJD26 is subsequently digested with PstI and SacII and the large fragment isolated. This fragment encodes all of a hybrid plasminogen activator except for ~23 residues surrounding $asp_{355}$ in the protease domain. This fragment is ligated to two oligonucleotides that anneal to each other to form a PstI-compatible sticky end on one end and a SacII-compatible sticky end on the other end. The duplex formed by these two oligonucleotides re-create the coding region for all of the residues that were encoded by the small PstI-SacII fragment that was removed from pJD26 except for the DNA encoding $asp_{355}$. This duplex contains DNA that encodes all possible amino acids at the position corresponding to $asp_{355}$. Thus, the oligonucleotides that form the duplex are actually degenerate mixtures that contain all possible codons at the position that previously encoded $asp_{355}$.

The ligation mix is used to transform E. coli. Bacterial colonies containing versions of pJD26 with DNA encoding different amino acids in place of $asp_{355}$ are scraped from plates and plasmid DNA prepared (a mixture of the different plasmids). This mixture of transfer vectors is used to produce a mixture of recombinant baculovirus expression vectors. Insect cells are then infected with these transfer vectors and four hours later washed in serum-free medium, overlayed with agarose containing serum-free culture medium, casein (prepared as in the casein plate assay) and plasminogen (1-20 μg/ml final concentration). Large clearing zones appear around some plaques that contain recombinant virus encoding an $asp_{355}$ substitution. The exact sequence of the expression vectors that encode the plasminogen activators with improved specific activity is determined by either direct sequencing of the isolated viral DNA or by first amplifying the DNA using the polymerase chain reaction method of U.S. Patent Nos. 4,683,195 and 4,683,202, issued 28 July 1987, both of which are assigned to Cetus Corporation and incorporated herein by reference.

### Deposits

The materials listed below were deposited with the American Type Culture Collection, Rockville, MD, USA (ATCC). The deposits were made under the provisions of the Budapest Treaty on the International Recognition of the Deposit of Microorganisms for the Purposes of Patent Procedure and the Regulations thereunder (Budapest Treaty). Maintenance of a viable culture is assured for 30 years from date of deposit. The organism will be made available by ATCC under the terms of the Budapest Treaty, and subject to an agreement between Applicants and ATCC which assures unrestricted availability upon issuance of the pertinent U.S. patent. Availability of the deposited strains are not to be construed as a license to practice the invention in contravention of the rights granted under the authority of any government in accordance with its patent laws.

| Recombinant Transfer Vector | CMCC# | ATCC# | Deposit Date |
|---|---|---|---|
| pPD7 (aka pPD7b) | 2929 | 67280 | 18 December 1986 |
| pPD8 (aka pPD8a) | 2928 | 67279 | 18 December 1986 |
| pPD18 | 2862 | 67431 | 12 June 1987 |
| pPD19 | 2863 | 47432 | 12 June 1987 |

The foregoing written specification is considered to be sufficient to enable one skilled in the art to practice the invention. The present invention is not to be limited in scope by the cell lines deposited, since the deposited embodiments are intended as illustrations of the invention. The deposit of materials therein does not constitute an admission that the written description herein contained is inadequate to enable the practice of any aspect of the invention, including the best mode thereof, nor are the deposits to be construed as limiting the scope of the claims to the specific illustrations that they represent. Indeed, various modifications of the invention would be appreciated by those skilled in the art from the foregoing description and fall within the scope of the appended claims.

**Claims**

1. A protein comprising urokinase modified at at least one amino acid wherein said modified urokinase is less susceptible than unmodified urokinase to cleavage in the region corresponding to lysine 158 in urokinase and a second modification of at least one amino acid wherein said second modification produces a modified urokinase with increased activity compared to a molecule having only said first modification.

2. The protein of claim 1 wherein at least one neutral or negatively charged amino acid is substituted for the amino acid residue oorresponding to lysine 158 in urokinase.

3. The protein of claim 1 or claim 2 wherein at least one neutral or positively charged amino acid is substituted for the amino acid residue corresponding to aspartic acid 355 in urokinase.

4. A recombinant plasminogen activator protein comprising a domain capable of interacting with fibrin and a protease domain wherein said protease domain comprises an amino acid sequence that is substantially the same as the protease domain of urokinase.

5. A recombinant plasminogen activator protein comprising at least one domain capable of interacting with fibrin and a protease domain wherein said protease domain comprises an amino acid sequence that is substantially the same as the protease domain of the modified urokinase of any one of claims 1 to 3, for example wherein the lysine corresponding to lysine 158 of urokinase is altered whereby said protease domain is resistant to cleavage at said lysyl residue and optionally wherein the aspartic acid corresponding to aspartic acid 355 of urokinase is altered whereby the altered protein has an increased activity compared to a protein having only said lysine 158 modification.

6. The protein of claim 4 wherein the domain capable of interacting with fibrin comprises an amino acid sequence selected from the group consisting of lysine-binding kringle domains and finger domains.

7. The protein of claim 6 wherein said finger domain is substantially the same as the finger region of tissue plasminogen activator.

8. The protein of claim 6 wherein said lysine-binding kringle domain is substantially the same as one selected from the group consisting of kringle-2 of t-PA, kringle-1 and kringle-4 of plasminogen.

9. The protein of claim 8 which further comprises a finger domain comprising an amino acid sequence that is substantially the same as the finger region of tissue plasminogen activator.

10. A DNA sequence encoding an amino acid sequence comprising the protein of any of claims 1 to 9.

11. A DNA sequence encoding a protein of any one of claims 4 to 9 in operable linkage with control sequences for the expression of said protein in a host cell.

12. A DNA sequence according to claim 11 wherein said control sequences are selected from those operable in prokaryotic cells, plant cells, mammalian cells, and insect cells.

13. An expression system comprising a prokaryotic cell or a mammalian cell or an insect cell transformed with the DNA sequence of claim 11.

14. A DNA sequence encoding a heterologous secretory signal peptide in proper translational reading frame with the DNA sequence of claim 11, wherein said heterologous secretory signal peptide is encoded by the DNA sequence.

5' CATGGCGTTAGCTATTACTCTTGCATTATTGCTTCTCCTACTGTTATTGCCTG
GGTGT

3' CGCAATCGATAATGAGAACGTAATAACGAAGAGGATGACAAT
AACGGACCCACA

TGGGCTCAACCCGG 3'
ACCCGAGTTGGGCCGTAC 5'

15. A method for producing a polypeptide with plasminogen activator activity comprising cotransfecting a susceptible host insect cell with a recombinant baculovirus transfer vector and baculovirus DNA wherein said transfer vector comprises at least one DNA sequence encoding the protein of any one of claims 1 to 9 under the transcriptional control of a vaculovirus promoter, isolating a recombinant baculovirus expression vector, infecting insect cells under suitable conditions with said isolated expression vector, and recovering said polypeptide from the culture medium.

16. A method of facilitating the secretion of a protein, which method consists of designing a secretory signal peptide comprising amino acid residues within the region encompassing positions -13 through +2 of said protein, wherein one or more of the residues are selected from among the four most frequently used residues at each selected postion as determined from a database of known secretory peptides, optionally the selection of said amino acid residues being fruther adjusted for the expected frequency of each residue.

17. The use of a protein of any one of claims 1 to 9 in the manufacture of a medicament.

5'......................OLIGODEOXYRIBONUCLEOTIDE..............................3'

A CC ATG GGA TGT TAT TTT GGA AAT GGA TCA GCC TAC CGT GGC ACG CAC AGG CTG ACC GAG

B GCT GTG CGT GCC ACG GTA GGC TGA TCC ATT TCC AAA ATA ACA TCC CAT GGG TAC

C AGC TTG GGC ACT GGG GTT CTG TGC TGT GTA AAC CTT GCC TAT CAG GAT CAT GGA ATT CCA CGG GAG GCA GGA GGC ACC CGA CTC GGT GAG

D TCG GGT GCC TCC TGC CTC CCG TGG AAT TCC ATG ATC CTG ATA GGC AAG GTT TAC ACA GCA CAG AAC CCC AGT GCC CA

E AGC TCT GGG CCT GGG CAA ACA TAA TTA CTG CCG GAA TCC TGA TGG GGA TGG CAA GCC CTG GTG CCA CGT GCT GAA GAA CCG CAG GCT GAC GTG GGA G

F C CTG CGG TTC TTC AGC ACG TGG CAC CAG GGC TTG GCA TCC CCA TCA GGA TTC CGG CAG TAA TTA TGT TTG CCC AGG CCC AG

G TAC TGT GAC GTC CCC TCC TGC TCC ACC TGC GGC CAA AAG ACT CTG CGG CCG CGC TTT GGT ATT ATT GGG GGA GAA TTC G

H TC GAC GAA TTC TCC CCC AAT AAT ACC AAA GCG CGG CCG CAG AGT CTT TTG GCC GCA GGT GGA GCA GGA GGG GAC GTC ACA GTA CTC CCA CGT CAG

I AGC TCC ATG GCG TTG TGC ATG CG

J GAT CCG CAT GCA CAA CGC CAT GG

K A GCT TGG TAC CGA TAT CGG ATC CTC AGA GGG CCA GGC CAT TCT CTT CCT TGG TGT GAC TTC T

L GA TCA GAA GTC ACA CCA AGG AAG AGA ATG GCC TGG CCC TCT GAG GAT CCG ATA TCG GTA CCA

FIG. 1

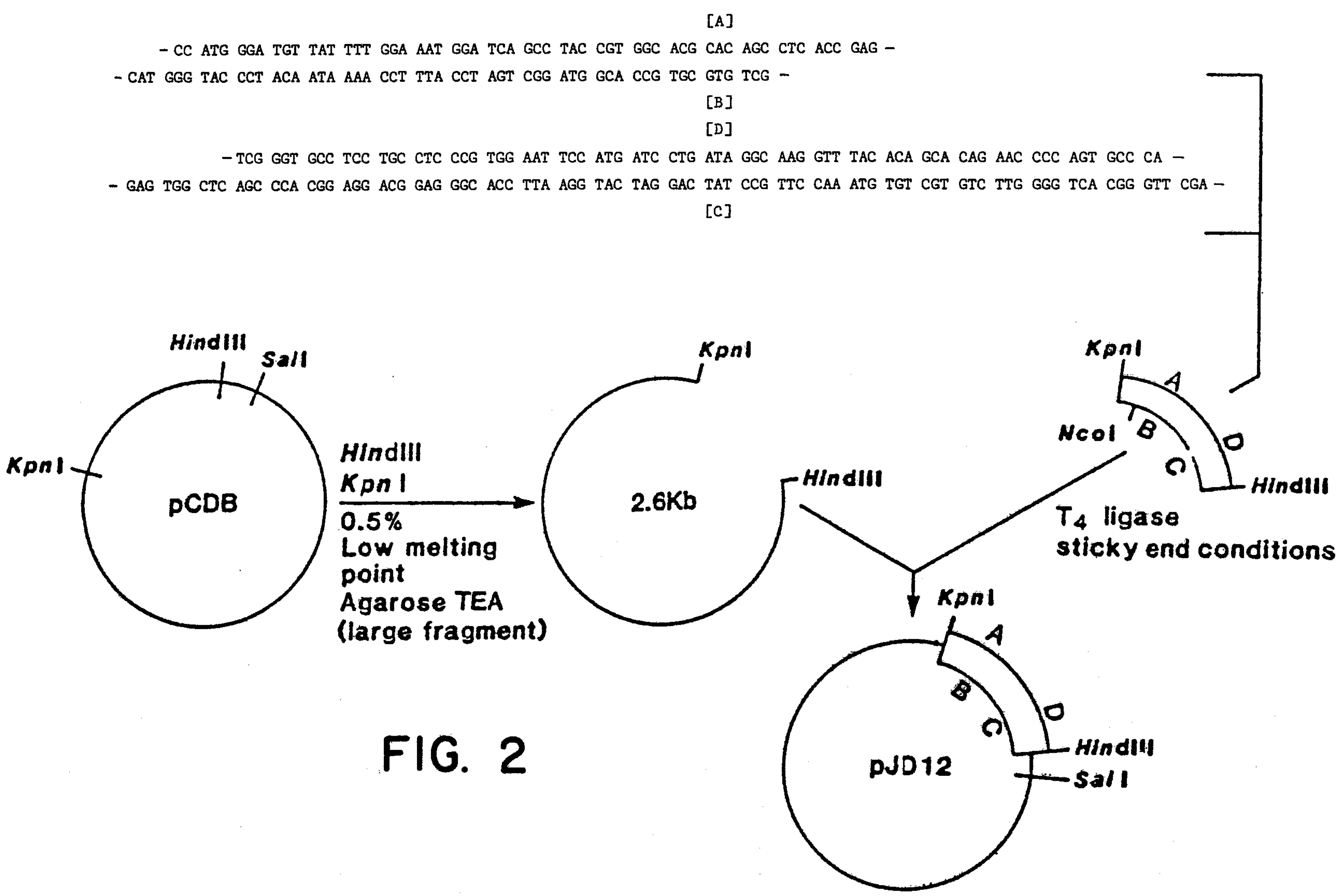
[A]
– CC ATG GGA TGT TAT TTT GGA AAT GGA TCA GCC TAC CGT GGC ACG CAC AGC CTC ACC GAG –
– CAT GGG TAC CCT ACA ATA AAA CCT TTA CCT AGT CGG ATG GCA CCG TGC GTG TCG –
[B]
[D]
– TCG GGT GCC TCC TGC CTC CCG TGG AAT TCC ATG ATC CTG ATA GGC AAG GTT TAC ACA GCA CAG AAC CCC AGT GCC CA –
– GAG TGG CTC AGC CCA CGG AGG ACG GAG GGC ACC TTA AGG TAC TAG GAC TAT CCG TTC CAA ATG TGT CGT GTC TTG GGG TCA CGG GTT CGA –
[C]
HindIII
SalI
KpnI
pCDB
HindIII
KpnI
0.5%
Low melting
point
Agarose TEA
(large fragment)
KpnI
2.6Kb
HindIII
KpnI
A
NcoI
B
C
D
HindIII
$T_4$ ligase
sticky end conditions
KpnI
A
B
C
D
HindIII
SalI
pJD12

FIG. 2

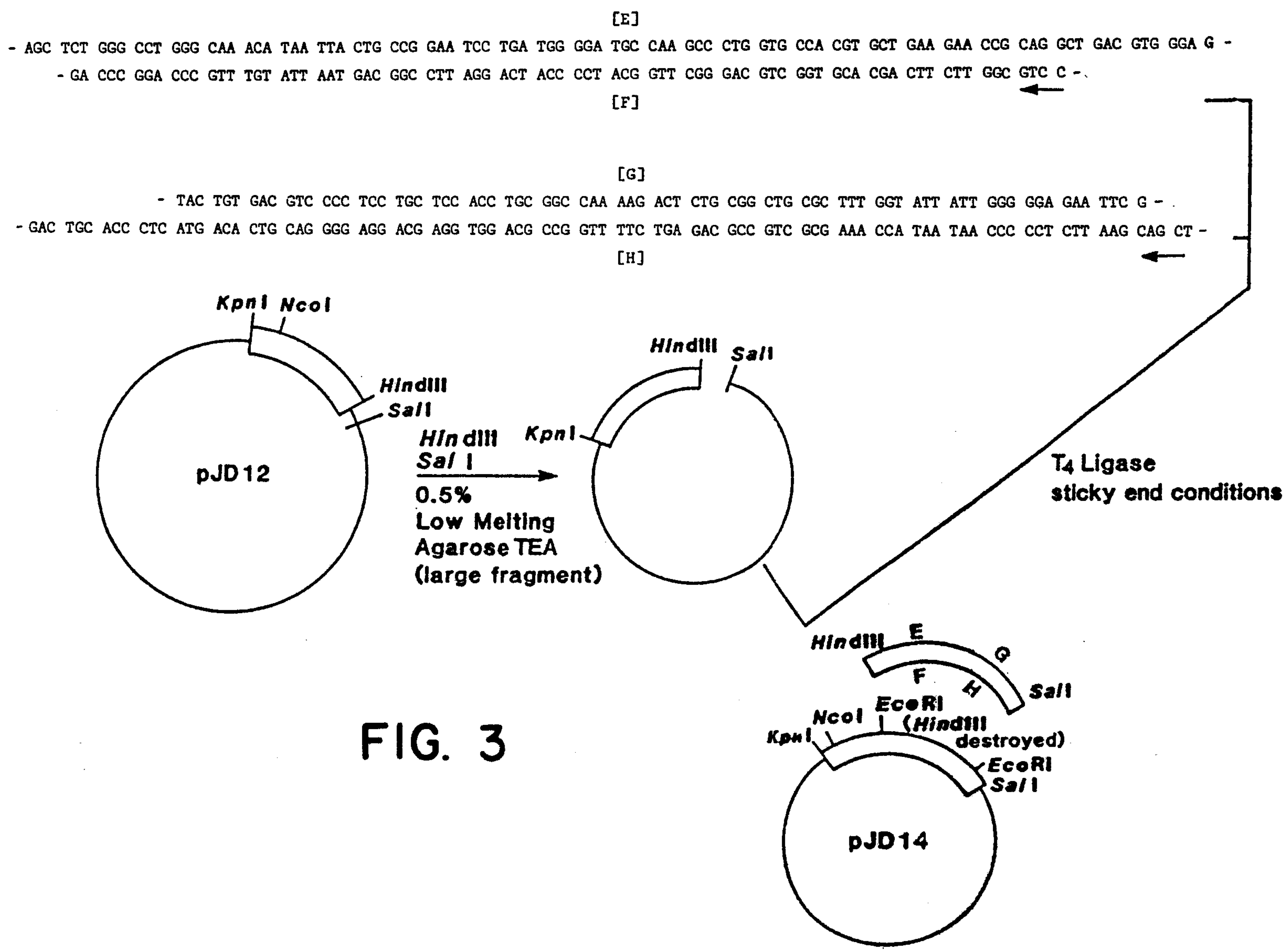
[E]
- AGC TCT GGG CCT GGG CAA ACA TAA TTA CTG CCG GAA TCC TGA TGG GGA TGC CAA GCC CTG GTG CCA CGT GCT GAA GAA CCG CAG GCT GAC GTG GGA G -
- GA CCC GGA CCC GTT TGT ATT AAT GAC GGC CTT AGG ACT ACC CCT ACG GTT CGG GAC GTC GGT GCA CGA CTT CTT GGC GTC C -
[F]
[G]
- TAC TGT GAC GTC CCC TCC TGC TCC ACC TGC GGC CAA AAG ACT CTG CGG CTG CGC TTT GGT ATT ATT GGG GGA GAA TTC G -
- GAC TGC ACC CTC ATG ACA CTG CAG GGG AGG ACG AGG TGG ACG CCG GTT TTC TGA GAC GCC GTC GCG AAA CCA TAA TAA CCC CCT CTT AAG CAG CT -
[H]
KpnI NcoI
HindIII
SalI
pJD12
HindIII
SalI
0.5%
Low Melting
Agarose TEA
(large fragment)
KpnI
HindIII
SalI
T4 Ligase
sticky end conditions
HindIII
E
F
G
H
SalI
KpnI
NcoI
EcoRI
(HindIII
destroyed)
EcoRI
SalI
pJD14

FIG. 3

# FIG. 4

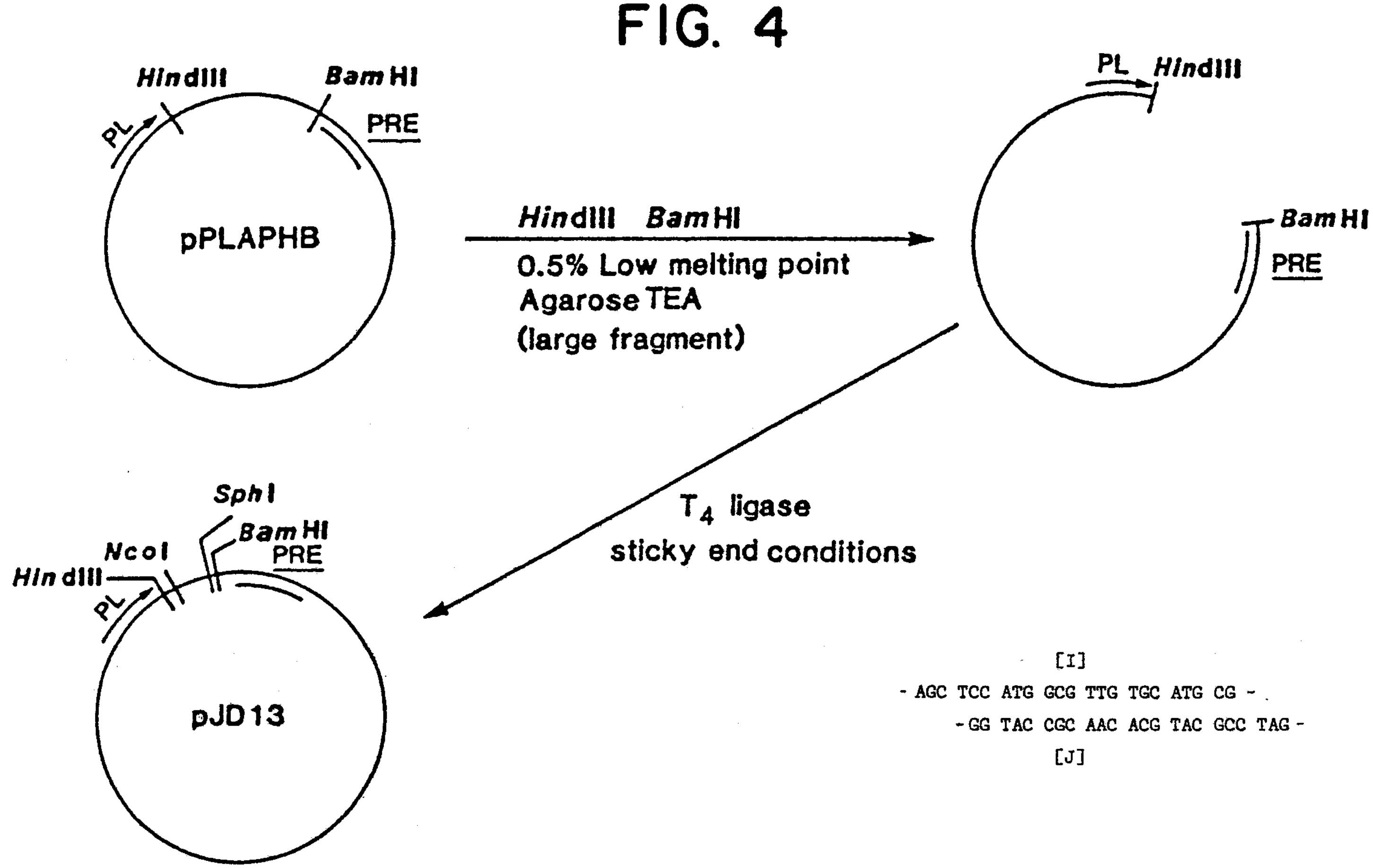


[I]
- AGC TCC ATG GCG TTG TGC ATG CG -
- GG TAC CGC AAC ACG TAC GCC TAG -
[J]

FIG. 5

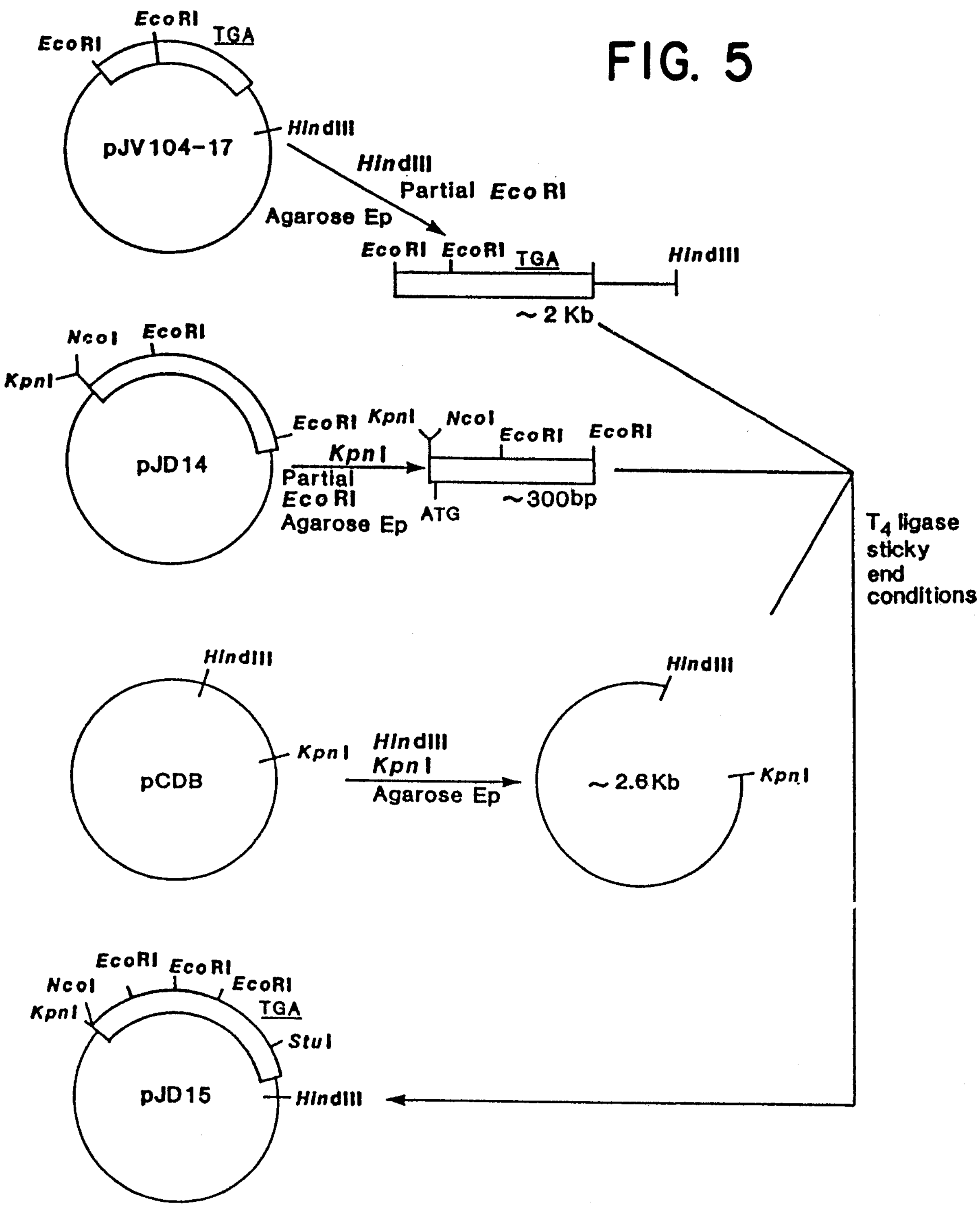
EcoRI
EcoRI
TGA
pJV104-17
HindIII
HindIII
Partial EcoRI
Agarose Ep
EcoRI EcoRI TGA
HindIII
~2 Kb
NcoI
EcoRI
KpnI
EcoRI
KpnI
NcoI
EcoRI
EcoRI
pJD14
KpnI
Partial
EcoRI
Agarose Ep
ATG
~300bp
T4 ligase
sticky
end
conditions
HindIII
HindIII
pCDB
KpnI
HindIII
KpnI
Agarose Ep
~2.6Kb
KpnI
EcoRI
EcoRI
NcoI
EcoRI
KpnI
TGA
StuI
pJD15
HindIII

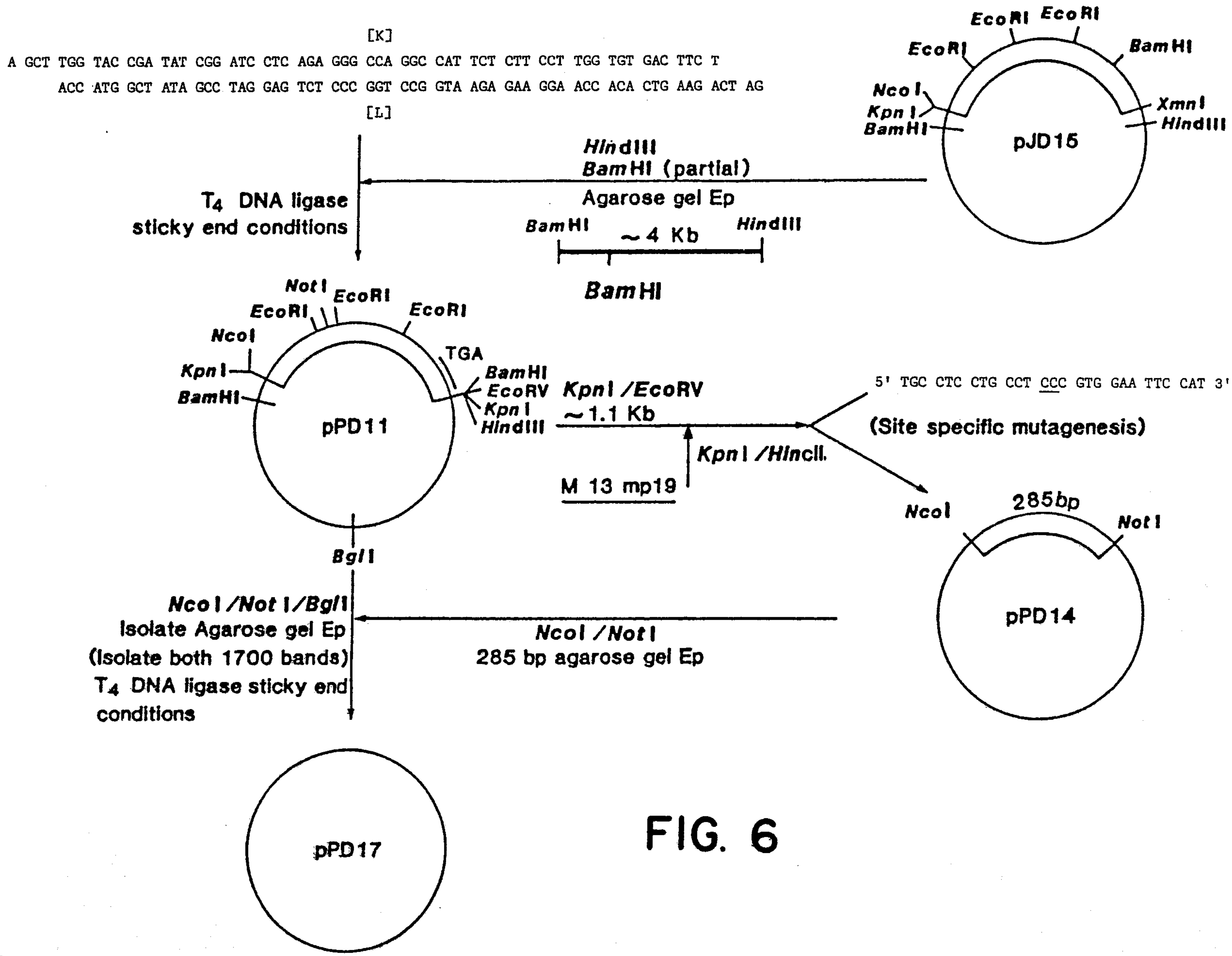
[K]
A GCT TGG TAC CGA TAT CGG ATC CTC AGA GGG CCA GGC CAT TCT CTT CCT TGG TGT GAC TTC T
ACC ATG GCT ATA GCC TAG GAG TCT CCC GGT CCG GTA AGA GAA GGA ACC ACA CTG AAG ACT AG
[L]
EcoRI
EcoRI
EcoRI
BamHI
NcoI
KpnI
BamHI
XmnI
HindIII
pJD15
HindIII
BamHI (partial)
Agarose gel Ep
BamHI
~4 Kb
HindIII
BamHI
T4 DNA ligase
sticky end conditions
NotI
EcoRI
EcoRI
EcoRI
NcoI
KpnI
BamHI
TGA
BamHI
EcoRV
KpnI
HindIII
pPD11
KpnI /EcoRV
~1.1 Kb
KpnI /HincII
M 13 mp19
5' TGC CTC CTG CCT CCC GTG GAA TTC CAT 3'
(Site specific mutagenesis)
NcoI
285bp
NotI
pPD14
BglI
NcoI /NotI /BglI
Isolate Agarose gel Ep
(Isolate both 1700 bands)
T4 DNA ligase sticky end
conditions
NcoI /NotI
285 bp agarose gel Ep
pPD17

FIG. 6

FIG. 7

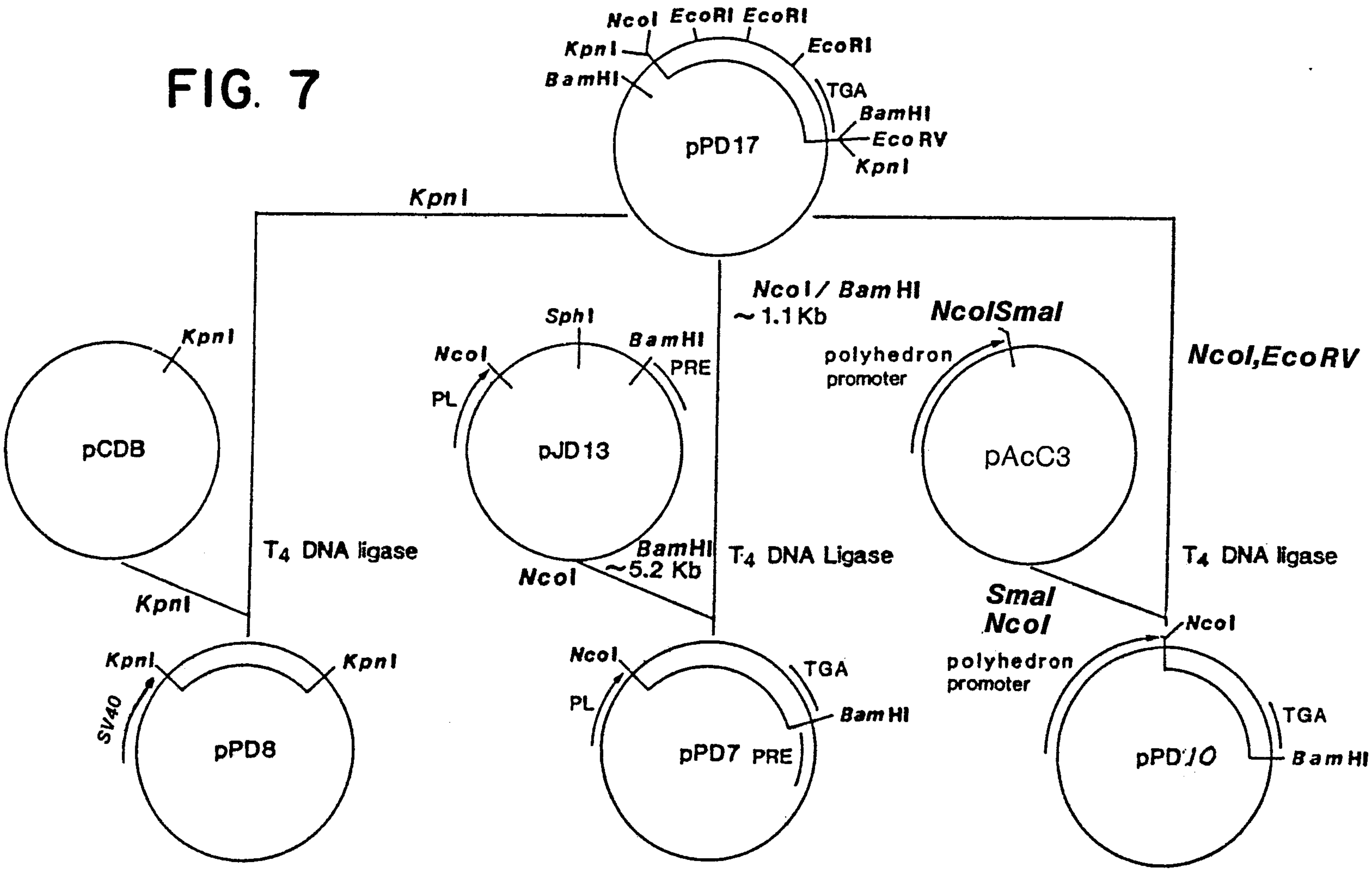
NcoI EcoRI EcoRI
KpnI
BamHI
EcoRI
TGA
BamHI
EcoRV
KpnI
pPD17
KpnI
NcoI / BamHI
~1.1 Kb
NcoISmaI
NcoI,EcoRV
KpnI
pCDB
SphI
BamHI
PRE
NcoI
PL
pJD13
polyhedron
promoter
pAcC3
T4 DNA ligase
BamHI
~5.2 Kb
NcoI
T4 DNA Ligase
T4 DNA ligase
SmaI
NcoI
KpnI
NcoI
polyhedron
promoter
KpnI
KpnI
SV40
pPD8
NcoI
TGA
PL
BamHI
pPD7 PRE
TGA
BamHI
pPD10

0273774

FIG. 8

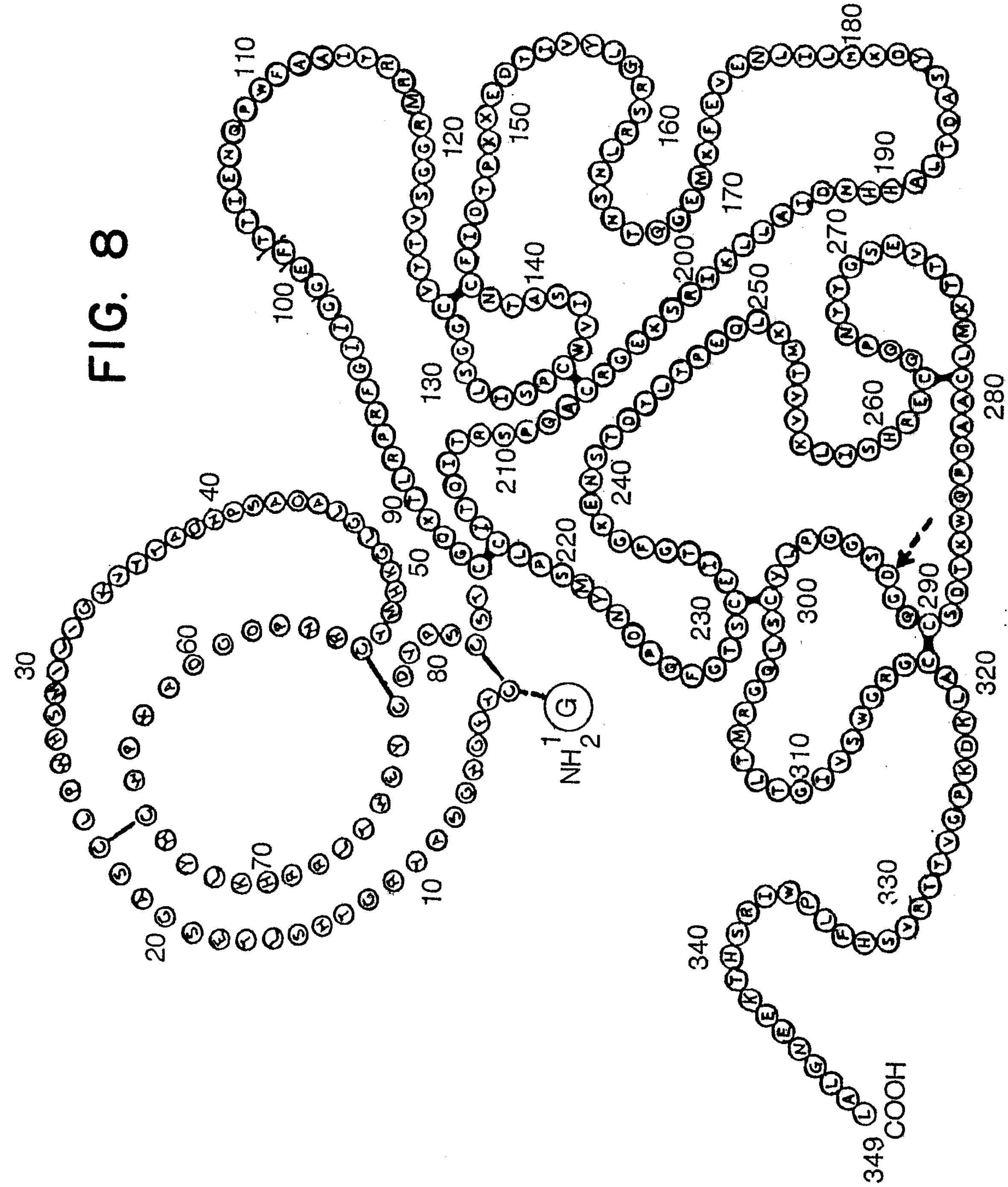

M 15.01.88

0273774

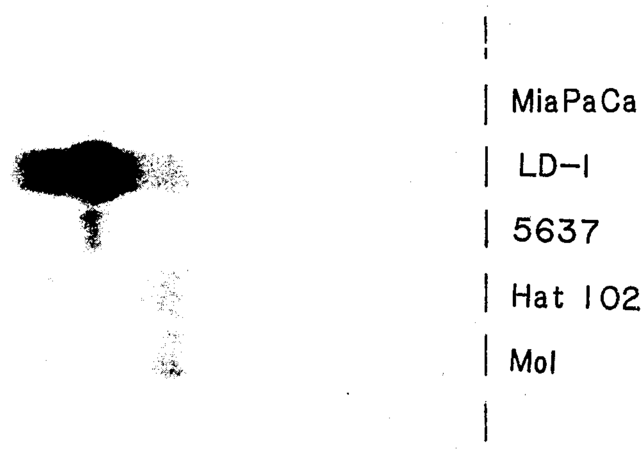


FIG. 9

0273774

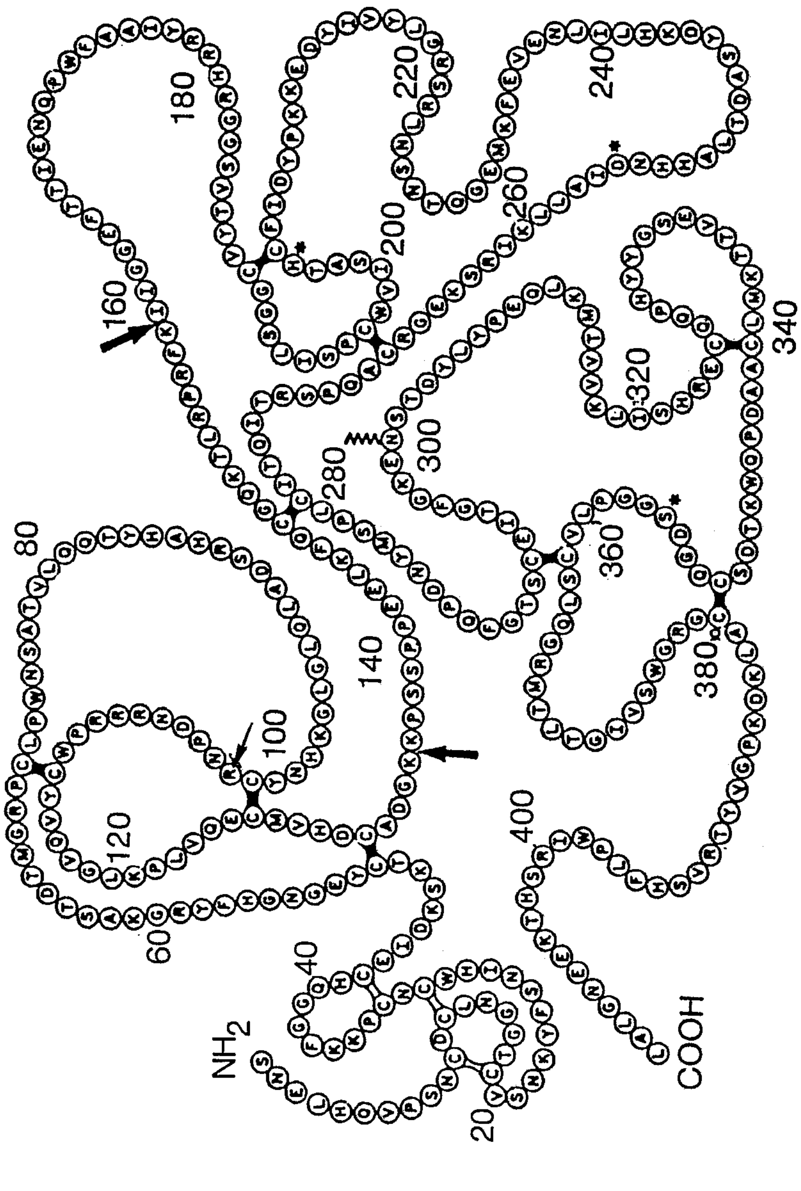


FIG. 10

FIG. 11

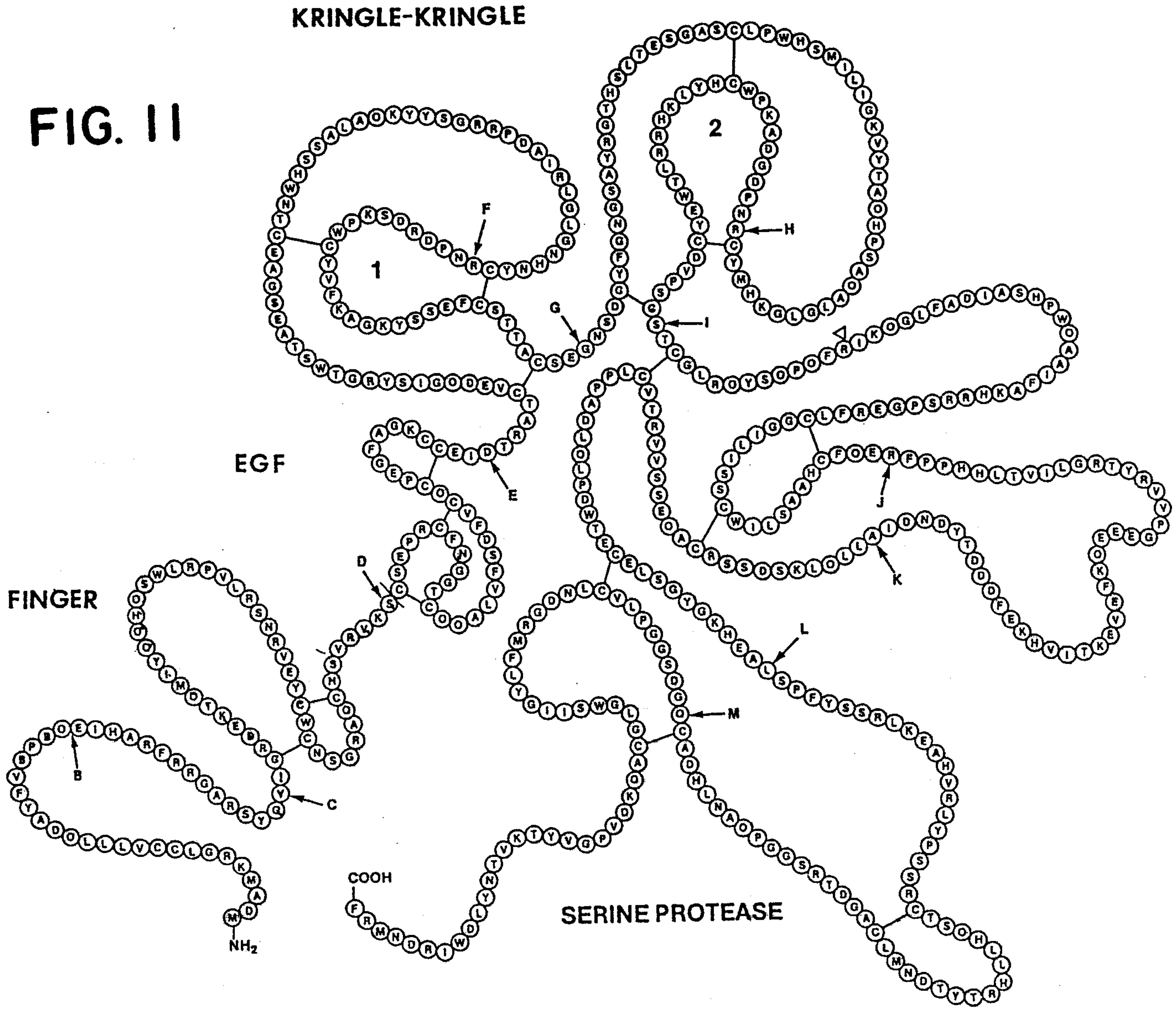
KRINGLE-KRINGLE
EGF
FINGER
SERINE PROTEASE
1
2
NH2
COOH
B
C
D
E
F
G
H
I
J
K
L
M

0273774

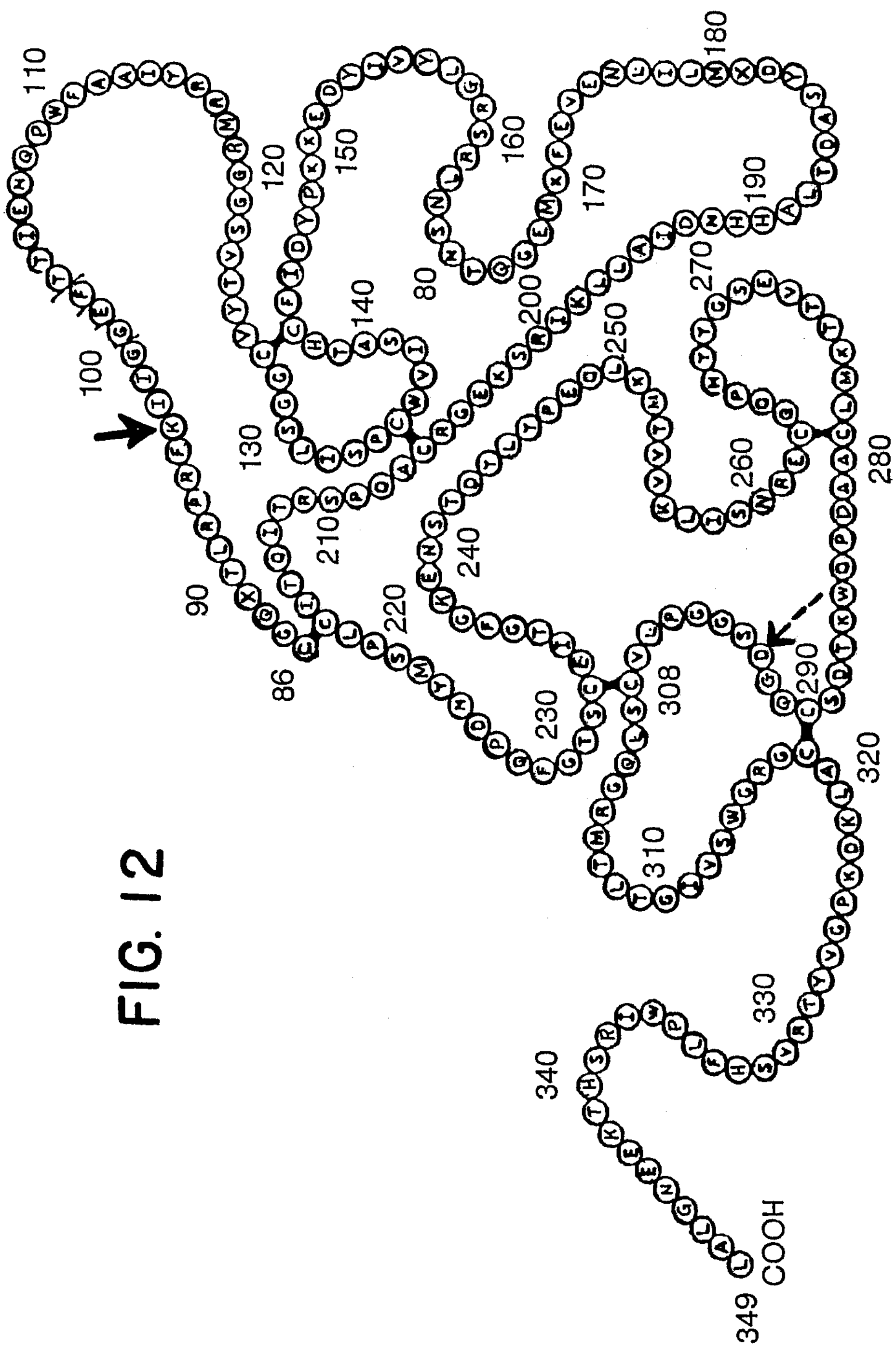


FIG. 12

```
   1 GGATGTTATTTTGGAAATGGATCAGCCTACCGTGGCACGCACAGCCTCACCGAGTCG
     GlyCysTyrPheGlyAsnGlySerAlaTyrArgGlyThrHisSerLeuThrGluSer

  60 GGTGCCTCCTGCCTCCCGTGGAATTCCATGATCCTGATAGGCAAGGTTTACACAGCACAG
     GlyAlaSerCysLeuProTrpAsnSerMETIleLeuIleGlyLysValTyrThrAlaGln

 120 AACCCCAGTGCCCAAGCTCTGGGCCTGGGCAAACATAATTACTGCCGGAATCCTGATGGG
     AsnProSerAlaGlnAlaLeuGlyLeuGlyLysHisAsnTyrCysArgAsnProAspGly

 180 GATGCCAAGCCCTGGTGCCACGTGCTGAAGAACCGCAGGCTGACGTGGGAGTACTGTGAC
     AspAlaLysProTrpCysHisValLeuLysAsnArgArgLeuThrTrpGluTyrCysAsp

 240 GTCCCCTCCTGCTCCACCTGCGGCCAAAAGACTCTGCGGCCGCGCTTTGGTATTATTGGG
     ValProSerCysSerThrCysGlyGlnLysThrLeuArgProArgPheGlyIleIleGly

 300 GGAGAATTCACCACCATCGAGAACCAGCCCTGGTTTGCGGCCATCTACAGGAGGCACCGG
     GlyGluPheThrThrIleGluAsnGlnProTrpPheAlaAlaIleTyrArgArgHisArg

 360 GGGGGCTCTGTCACCTACGTGTGTGGAGGCAGCCTCATCAGCCCTTGCTGGGTGATCAGC
     GlyGlySerValThrTyrValCysGlyGlySerLeuIleSerProCysTrpValIleSer

 420 GCCACACACTGCTTCATTGATTACCCAAAGAAGGAGGACTACATCGTCTACCTGGGTCGC
     AlaThrHisCysPheIleAspTyrProLysLysGluAspTyrIleValTyrLeuGlyArg

 480 TCAAGGCTTAACTCCAACACGCAAGGGGAGATGAAGTTTGAGGTGGAAAACCTCATCCTA
     SerArgLeuAsnSerAsnThrGlnGlyGluMETLysPheGluValGluAsnLeuIleLeu

 540 CACAAGGACTACAGCGCTGACACGCTTGCTCACCACAACGACATTGCCTTGCTGAAGATC
     HisLysAspTyrSerAlaAspThrLeuAlaHisHisAsnAspIleAlaLeuLeuLysIle

 600 CGTTCCAAGGAGGGCAGGTGTGCGCAGCCATCCCGGACTATACAGACCATCTGCCTGCCC
     ArgSerLysGluGlyArgCysAlaGlnProSerArgThrIleGlnThrIleCysLeuPro

 660 TCGATGTATAACGATCCCCAGTTTGGCACAAGCTGTGAGATCACTGGCTTTGGAAAAGAG
     SerMETTyrAsnAspProGlnPheGlyThrSerCysGluIleThrGlyPheGlyLysGlu

 720 AATTCTACCGACTATCTCTATCCGGAGCAGCTGAAAATGACTGTTGTGAAGCTGATTTCC
     AsnSerThrAspTyrLeuTyrProGluGlnLeuLysMETThrValValLysLeuIleSer

 780 CACCGGGAGTGTCAGCAGCCCCACTACTACGGCTCTGAAGTCACCACCAAAATGCTGTGT
     HisArgGluCysGlnGlnProHisTyrTyrGlySerGluValThrThrLysMETLeuCys

 840 GCTGCTGACCCACAGTGGAAAACAGATTCCTGCCAGGGAGACTCAGGGGGACCCCTCGTC
     AlaAlaAspProGlnTrpLysThrAspSerCysGlnGlyAspSerGlyGlyProLeuVal

 900 TGTTCCCTCCAAGGCCGCATGACTTTGACTGGAATTGTGAGCTGGGGCCGTGGATGTGCC
     CysSerLeuGlnGlyArgMETThrLeuThrGlyIleValSerTrpGlyArgGlyCysAla.

 960 CTGAAGGACAAGCCAGGCGTCTACACGAGAGTCTCACACTTCTTACCCTGGATCAGAAGT
     LeuLysAspLysProGlyValTyrThrArgValSerHisPheLeuProTrpIleArgSer

1020 CACACCAAGGAAGAGAATGGCCTGGCCCTCTGA
     HisThrLysGluGluAsnGlyLeuAlaLeu
```

FIG. 13

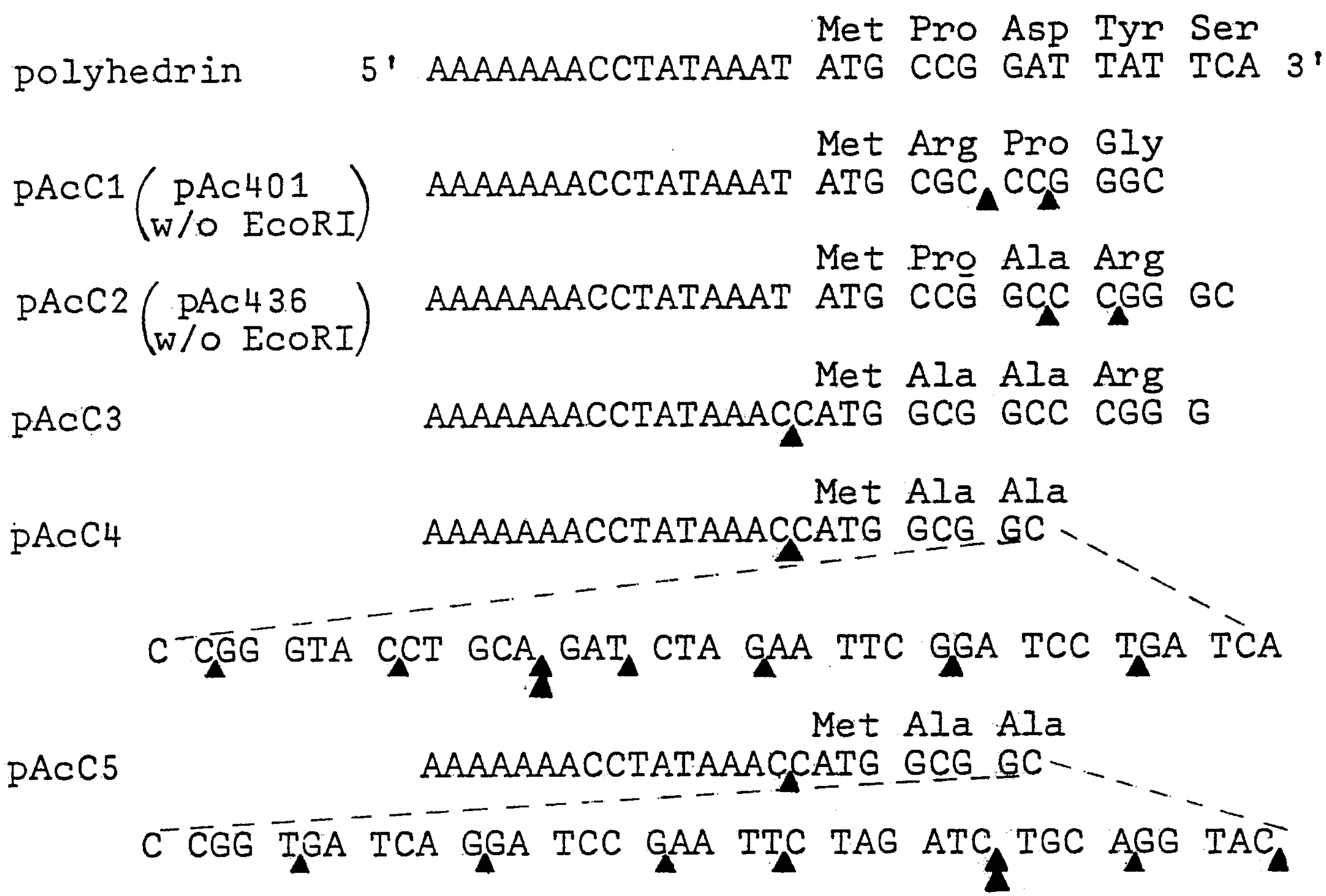


**BACULOVIRUS TRANSFER VECTORS**

**FIG. 14**